# EUROPÄISCHE PATENTSCHRIFT

(11) **EP 2 447 694 B1**
(45) Veröffentlichungstag und Bekanntmachung des Hinweises auf die Patenterteilung: **21.05.2014**
(21) Anmeldenummer: 10189276.8
(22) Anmeldetag: 28.10.2010
(51) Int. Cl.: G01M 3/20, G01M 3/00

(54) **Prüfleck zur Überprüfung von Leckagemesssystemen**
Test leak for inspecting leak measurement systems
Fuite d'essai pour le contrôle de systèmes de mesure de fuites

(43) Veröffentlichungstag der Anmeldung: 02.05.2012
(73) Patentinhaber: Boehringer Ingelheim Pharma GmbH & Co. KG, 55216 Ingelheim am Rhein (DE)
(72) Erfinder: Mathe, Gerald, 55216, INGELHEIM AM RHEIN (DE); Hahn, Christoph, 55216, INGELHEIM AM RHEIN (DE); Ziegler, Jochen, 55216, INGELHEIM AM RHEIN (DE); Scaffifi, Luigi, 55216, INGELHEIM AM RHEIN (DE)
(74) Vertreter: Simon, Elke Anna Maria

(56) Entgegenhaltungen:
- DE-A1- 2 926 112
- DE-U1- 29 810 978
- US-A- 3 209 579

## Beschreibung

Die vorliegende Erfindung betrifft eine Vorrichtung zur Überprüfung von Leckagemesssystemen oder gasanalytischen Messsystemen und ein Verfahren zur Überprüfung von Leckagemesssystemen oder gasanalytischen Messsystemen. Insbesondere handelt es sich bei der Vorrichtung um ein so genanntes Prüfleck. Die Erfindung bezieht sich insbesondere auf Leckagemesssysteme, die bei der Dichtigkeitsprüfung von Gegenständen verwendet werden, in denen sich Flüssigkeit befindet. Vorzugsweise sind diese Messsysteme derart aufgebaut, dass der zu prüfende Gegenstand in eine geschlossene Kammer eingesetzt wird, wo austretendes Gas oder austretende Flüssigkeitsbestandteile über einen vorbestimmten Zeitraum gesammelt wird. Anschließend werden das gesammelte Gas oder die gesammelten Flüssigkeitsbestandteile dann mittels eines Trägergasstroms einem auf dieses Gas oder diesen Flüssigkeitsbestandteil reagierenden Sensor oder einem Messsystem mit selektiver Substanzerkennung zugeführt.

Aus dem Stand der Technik ist eine Vielzahl von Leckagemesssystemen oder Verfahren zur Dichtigkeitsmessung bekannt.

Häufig werden Behälter ohne feste oder flüssige Befüllung auf Dichtigkeit geprüft, indem der abgedichtete Behälter mit einem Prüfgas wie beispielsweise Helium befüllt und in eine Prüfkammer eingebracht wird. In der Prüfkammer wird ein Vakuum erzeugt, und das aus dem Behälter austretende Gas wird entweder einem selektiven Sensor oder einem selektiven Messsystem wie beispielsweise einem Massenspektrometer zugeleitet, als Probe in ein Messsystem wie beispielsweise einem Gaschromatographen eingespritzt oder bereits durch den resultierenden Druckanstieg an einem Drucksensor in der Testkammer erfasst.

Im Spezialfall von mit Druckgas oder mit einem Gemisch aus Flüssigkeit und gasförmigen Treibmittel befüllten Dosen wie beispielsweise den Aerosol-Behältern aus den treibgasunterstützten medizinischen Zerstäubern, die im Englischen als "Metered Dose Inhaler" (kurz MDI) bezeichnet werden, verläuft die Prüfung analog, wobei das inherente Druckgas die Funktion des extra eingebrachte Prüfgases übernimmt. Die US4045996 offenbart eine Testvorrichtung für Leckdetektoren in der Produktionslinie solcher Dosen. Die Testvorrichtung umfasst eine Dose mit einer definierten Öffnung, die mit Filterpapier mit definierter Porosität verschlossen ist. Die Testvorrichtung ist derart mit unter Druck stehendem Fluid gefüllt, dass durch die mit Filterpapier verschlossene Öffnung ein wenig mehr Treibmittel austritt, als es bei der Dosenproduktion zulässig ist. Der übliche Druck solcher Dosen beträgt 3 bis 4 bar.

Die US 3956923 offenbart ein Testverfahren von Aerosol-Behältern auf kleine Leckagen, bei dem eine oder mehr Testkammern mit den zu prüfenden Aerosol-Behältern genau wie eine Kalibrierkammer mit einem Leckage-Standard, d.h. einem Behälter bekannter Leckage, evakuiert werden. Nach einer vorbestimmten Zeit werden die Kammern mit Trägergas gespült. Nach Erreichen des Mischungsgleichgewichtes der Gase in den Kammern werden Gasproben abwechselnd aus Testkammer und Kalibrierkammer auf einen Detektor oder durch ein Messgerät geleitet. Das Messsystem beinhaltet Vergleichsmöglichkeiten, so dass alle Aerosol-Behälter mit größeren Leckageraten als der Leckage-Standard in der Kalibrierkammer z.B. zwecks Aussortierung identifiziert werden. Alternativ zur Kalibrierkammer mit Leckage-Standard wird auch der Einsatz einer einstellbaren, Druck regulierten Quelle des Treibmittels beschrieben. Hierbei wird eine an einen Gasvorrat angeschlossene Kapillaröffnung beschrieben, deren Temperatur durch eine Heizung gesteuert wird.

Größere mit Prüfgas befüllte Prüflinge werden häufig mit einer so genannten Schnüffelspitze abgetastet anstatt in eine Prüfkammer eingebracht zu werden. Die Schnüffelspitzen nehmen das aus einem eventuell vorhandenen Leck ausströmende Testgas auf und führen es einem Testgasdetektor zu. Solche Testgas-Lecksuchgeräte werden mittels solcher Prüflecks kalibriert, die einen Gasvorrat und eine Engstelle mit bekanntem Leitwert aufweisen und aus denen während des Kalibrierens eine definierte Testgasrate ausströmt. Der Gasvorrat befindet sich hierbei meist in einer Druckdose, die das gewünschte Testgas in verflüssigter Form enthält.

In einem anderen Verfahren, mit dem auch befüllte Behälter getestet werden können, werden diese, wie in der WO2004/034009 A1 offenbart, von außen mit einem Prüfgas beaufschlagt und das Behälterinnere wird anschließend auf Vorhandensein des Prüfgases untersucht.

Meistens werden bei der Dichtigkeitsprüfung von flüssigbefüllten Behältern jedoch Wägeverfahren verwendet: Über einen langen Zeitraum, meist mindestens mehrere Tage oder gar Wochen, werden die zu prüfenden Behälter gewogen und ihr Gewicht mit den bei den vorherigen Messungen erzielten Werten verglichen. Auf diese Weise wird ein Masseverlust ermittelt, aus dem der Flüssigkeitsverlust oder eine Leckagerate bestimmt wird.

Die DE 103 16 332 A1 beschreibt ein Verfahren zur Prüfung der Dichtigkeit von Produkten, in die ein Fluid mit eingeschlossen ist. Das Produkt wird in eine Prüfkammer eingesetzt, in der ohne äußere Unterdruckeinwirkung ein austretender Fluidbestandteil über einen vorbestimmten Zeitraum gesammelt wird und dann mittels eines der Kanalisierung dienenden Trägergasstroms einem Sensor zugeführt wird. Der Sensor reagiert auf den Fluidbestandteil, das Fluid wird quantitativ erfasst und ein ermittelter Wert mit einem Grenzwert verglichen. Während des Zeitraums, in dem der aus dem Produkt austretende Fluidbestandteil in der Prüfkammer gesammelt wird, wird der Trägergasstrom über einen Bypass dem Sensor zugeleitet.

Typischerweise erfolgt die Kalibrierung solcher mit Gassensoren arbeitenden Leckagemesssystemen mit einer Kalibriergasquelle: Ein Kalibriergasstrom mit bekannter Gaskonzentration wird entweder direkt auf den Sensor geleitet oder der Messkammer zugeführt, wo es durch Durchflussregler gesteuert konstant an die Stärke des Trägergasstroms angepasst wird.

Die DE 29810978 U1, US3209579 A und DE 2926112 A1 offenbaren ein Prüfleck zur Überprufüng von Leckagemesssystemen.

Der vorliegenden Erfindung liegt die Aufgabe zugrunde, ein gegenüber dem Stand der Technik verbessertes Verfahren zur Überprüfung und Kalibrierung von gasanalytischen Messsystemen oder Leckagemesssystemen anzugeben. Im Fall der beabsichtigten Überprüfung von flüssigbefüllten Gegenständen oder Behältern soll eine hierzu geeignete Prüfvorrichtung in Form eines Prüflecks angegeben werden, das zuverlässig und konstant eine bekannte Leckage aufweist. Mit Hilfe dieses Prüflecks soll das jeweilige Messsystem unter anwendungsnahen Bedingungen getestet werden können. Insbesondere soll die Messsituation, dass gasförmige oder von Gas transportierte aus einem Gegenstand austretenden Flüssigkeitsbestandteile zu bestimmen sind, dabei wiedergespiegelt werden. Mit Hilfe solcher Prüflecks sollen Leckagemesssysteme auch auf ihre Verwendbarkeit bei Dichtigkeitsprüfungen von flüssigbefüllten Behältern getestet und von ihnen generierte Signale oder Messwerte quantitativ auswertbar gemacht werden können.

Die genannte Aufgabe wird erfindungsgemäß gelöst durch ein Flüssigkeit enthaltenes Prüfleck, aus dem Gas oder Dampf oder von Gas transportierte Flüssigkeitsbestandteile austreten, wobei dieses Gas oder dieser Dampf oder diese Bestandteile durch den der Flüssigkeit eigenen Dampfdruck durch eine Festkörperschicht gebildet werden, wobei die Flüssigkeit in einem Speichermaterial eingelagert oder gebunden oder tamponiert ist und die Flüssigkeit keinen direkten Kontakt zu dem Bereich der Festkörperschicht aufweist. Das Prüfleck dient dem Einsatz in einem gasanalytischen Messsystem oder einem Leckagemesssystem oder der Überprüfung eines gasanalytischen Messsystem oder Leckagemesssystems.

Des Weiteren wird die erfindungsgemäße Aufgabe gelöst durch ein Verfahren zur Überprüfung oder Kalibrierung eines gasanalytischen Messsystems oder eines Leckagemesssystems, dass sich dadurch kennzeichnet dass ein erfindungsgemäßes Prüfleck zur Signalerzeugung oder zum Test auf das Vorhandensein eines Signals im Messsystem verwendet wird oder mehrere unterschiedlich ausgasende Prüflecks zur Erfassung eines Datensatzes im Messsystem verwendet werden.

Vorteilhafte Weiterbildungen werden im Folgenden und im Detail anhand der Figuren beschrieben.

Das Prüfleck beinhaltet eine als Verdunstungsbarriere dienende Festkörperschicht, insbesondere eine Membran oder Folie, bevorzugt eine Polymerfolie, welche die Permeation von aus der Flüssigkeit gebildetem Gas oder Dampf oder von Molekülen aus der Flüssigkeit durch die Festkörperschicht hindurch zulässt und/oder an der das Gas oder der Dampf aus dem Prüfleck austritt. Auf diese Weise können bereits kleine Mengen austretenden Gases oder durch Leckage einem Gegenstand entweichende Substanzmengen nachgestellt werden. Darüber hinaus ist der hier genutzte Leckageweg über Permeations- und Diffusionsprozesse ein in sofern sehr realitätsnaher, als dass gerade solche Prozesse häufig diejenigen Leckagen verursachen, die mit Leckagemesssystemen an den zu prüfenden, Flüssigkeit beinhaltenden Gegenständen nachgewiesen werden sollen. Die Verwendung solcher Prüflecks in einem Messsystem entspricht also der für zu prüfende Gegenstände üblichen Messsituation.

Ein Merkmal eines erfindungsgemäßen Prüflecks ist, dass zumindest bei seiner Verwendung die Flüssigkeit keinen direkten Kontakt zu dem Bereich der Festkörperschicht aufweist, durch den Gas oder Flüssigkeitsbestandteile austreten kann. Insbesondere grenzt an die dem Inneren des Prüflecks zugewandten Oberfläche der Festkörperschicht ein Gas gefüllter Raum an, wobei das Gas entweder dem aus dem Prüfleck austretenden Gas bereits entspricht oder in Form einer Mischung mit diesem gesättigt ist. Ein solcher Aufbau hat den Vorteil, dass es nicht z.B. durch Flüssigkeitsbewegung zu wechselnden Ausgangsbedingungen für den an der Festkörperschicht stattfindenden Permeationsprozess kommen kann. Auf diese Weise wird eine gleichbleibende Permeation in der Festkörperschicht und somit eine hohe Reproduzierbarkeit und Konstanz der Leckagerate des Prüflecks sichergestellt. Bevorzugt wird dieser Effekt dadurch erzielt, dass die Flüssigkeit im Prüfleck in einem Speichermaterial, insbesondere in einem Schwamm oder Filtermaterial, eingelagert, gebunden oder tamponiert ist und/oder dass sich im Inneren des Prüflecks ein mit der Flüssigkeit benetztes Speichermaterial befindet. Dies trägt zur Gleichmäßigkeit der Leckage des Prüflecks, zur besseren Rückhaltung der Flüssigkeit und somit zu einer höheren Langzeitstabilität des Prüflecks bei. Zum Zweck schneller und einfacher Montage beinhaltet der Aufbau des Prüflecks vorzugsweise ein Gefäß mit einem Hohlraum, der die Flüssigkeit und/oder das Speichermaterial aufnimmt, wobei das Gefäß nur an einer Seite geöffnet ist und diese Öffnung direkt mit der die Permeation zulassenden Festkörperschicht verschlossen ist. Die Flüssigkeit im Prüfleck oder das Prüfleck insgesamt beinhaltet zwecks einfacherer Befüllung und Handhabbarkeit und meist auch zwecks Anpassung an die Messsituation keinerlei Zusätze von Druckgas oder Treibmitteln.

Ein Merkmal des Prüflecks und des erfindungsgemäßen Verfahrens ist, dass das Prüfleck hinsichtlich seines Aufbaus und seiner Eigenschaften an den jeweiligen mit einem Leckagemesssystem zu prüfenden Gegenstand anpassbar ist. Für die Eignungsprüfung eines Leckagemesssystems für seinen Einsatz bei der Dichtigkeitsprüfung eines Flüssigkeit beinhaltenden Gegenstandes wird so erfindungsgemäß ein Prüfleck verwendet, das die gleiche Flüssigkeit oder das gleiche flüssige Lösemittel oder den gleichen flüchtigen Bestandteil wie der auf Dichtigkeit zu prüfende Gegenstand enthält. Je nach Flüssigkeitsbefüllung des zu prüfenden Gegenstandes wird das Prüfleck mit der entsprechenden Flüssigkeit befüllt und die Leckagestelle am Prüfleck so ausgelegt, das eine Leckage der zum Beispiel vor dem Hintergrund von für den jeweiligen Gegenstand einzuhaltenden Grenzwerten oder einem anderen gewünschten Wert entspricht. Hierzu wird die die Leckagestelle bildende Festkörperschicht so gewählt, dass das Material und seine Dicke eine Permeation des für den Austritt vorgesehenen Gases oder Flüssigkeitsbestandteile zulässt. Eine geeignete Materialpaarung wie beispielsweise die Wahl einer Festkörperschicht aus Polyethylen zur Permeation von Ethanol wird hierbei beachtet. Durch die Anpassbarkeit des Prüflecks an den zu prüfenden Gegenstand wird die Plausibilität des Verfahrens zur Eignungsprüfung eine Messsystems zur Überprüfung der Dichtigkeit des zu prüfenden Gegenstands gewährleistet.

Ein weiteres Merkmal des erfindungsgemäßen Verfahrens ist, dass das Prüfleck eine Verdunstungsrate oder eine Gasaustrittsrate in einer solchen Höhe aufweist oder in einer Testkammer des Leckagemesssystems eine Gaskonzentration in solcher Höhe erzeugt, wie sie gemäß der Auslieferbedingungen des Gegenstandes für diesen nicht zulässig ist oder dem für den Gegenstand festgelegtem die Grenzwert entspricht. Auf diese Weise kann das Prüfleck beispielsweise zur Kontrolle der im Rahmen einer Warenausgangsprüfung stattfindenden Dichtigkeitsuntersuchung am zu prüfenden Gegenstand dienen. Das Prüfleck mit grenzwertiger Leckagerate kann identisch wie der normalerweise zu prüfende Gegenstand der Dichtigkeitsprüfung unterzogen werden, wobei ein korrekt arbeitendes Messsystem das Prüfleck als grenzwertigen Gegenstand erkennt. Eine schnelle, in Routineprozessen oder maschinellen Produktionsprozessen umsetzbare Funktionsprüfung des eingesetzten Messsystems wird ermöglicht, ohne dass es durch die Überprüfung zu Störungen im Produktionsablauf kommt.

Vorzugsweise werden auch die äußeren Abmessungen des Prüflecks an die des zu prüfenden Gegenstandes angepasst, so dass in der Prüfumgebung, beispielsweise in einer Testkammer bei gleicher ursächlicher Leckagerate vergleichbare Gaskonzentrationen erzielt werden. Des Weiteren kann ein Prüfleck mit ähnlichen Abmessungen wie der zu prüfende Gegenstand, in die gleichen maschinellen Abläufe wie dieser eingebunden werden. So kann zum Beispiel eine Überprüfung eines vollautomatisch arbeitenden Messsystems so ablaufen, dass das Prüfleck ohne teure Umrüstzeiten anstelle eines zu prüfenden Gegenstandes in die im Rahmen der Produktion oder Produktkontrollen ablaufenden Prozesse eingeschleust werden kann. Die Überprüfung des Messsystems dauert in diesem Fall gerade einmal genauso lang wie eine Routinemessung. Hierdurch erzielt man einen deutlichen Zeitgewinn und eine gut überprüfbare Zuverlässigkeit des Messsystems. Insbesondere entspricht das äußere Gesamtvolumen des Prüflecks dem jeweiligen Wert des zu prüfenden Gegenstands. Kann dies nicht realisiert werden, werden die Abweichungen zwischen den äußeren Volumina von Prüfleck und zu prüfendem Gegenstand bei der Auswertung der durch den zu prüfenden Gegenstand im Leckagemesssystem erzeugten Signale in Form einer Umrechnung berücksichtigt. Auf diese Weise wird insbesondere in Kombination mit der Kalibrierung des Messsystems mittels mehrerer verschieden ausgasenden Prüflecks eine quantitative Auswertbarkeit der mit dem Messsystem erzeugten Werte sichergestellt. Vorzugsweise ist das Prüfleck so aufgebaut, dass es die Leckage im dafür vorgesehenen Bereich der Festkörperschicht und insbesondere nur dort zulässt und dass das Prüfleck ansonsten überall dicht ist. Insbesondere kann nirgendwo aus dem Prüfleck Flüssigkeit austreten. Durch die Begrenzung der Leckage auf eine Stelle bzw. einen Bereich (auch Permeationsöffnung genannt) kann das Prüfleck auch gerichtet eingesetzt werden, z.B. bei der Überprüfung von so genannten Schnüffelspitzen oder anderen Leckagemesssystemen ohne Testkammer. Hierzu wird beispielsweise die Schnüffelspitze an die Permeationsöffnung eines Prüflecks herangeführt und das Erkennen des Gasaustritts dient als Funktionstest der Schnüffelspitze. Die Permeationsöffnung des Prüflecks kann hierbei beispielsweise die Form einer einzelnen Bohrung über einer Permeationsfläche haben. Bei der Überprüfung von Schnüffelspitzen, die der Erkennung von großen Lecks dienen, ist es zweckmäßig, Prüflecks mit ähnlich hohen Leckageraten zu verwenden.

Unter der Bezeichnung Prüfleck ist eine Vorrichtung zu verstehen, die eine insbesondere zu Prüfzwecken einsetzbare, definierte Leckage und/oder Leckagerate aufweist.

Die Verdunstung oder Leckage am erfindungsgemäßen Prüfleck entsteht vorzugsweise durch Diffusion oder Permeation einer innen befindlichen gasförmigen oder vorzugsweise flüssigen Substanz. Als Permeation wird der Stofftransport von Substanzen durch ein Material bezeichnet, wobei die treibende Kraft des Prozesses der Konzentrationsgradient des permeierenden Stoffes zwischen beiden Seiten des Materials ist. Daher ist der Permationskoeffizient von dem Diffusionsfluss der permeierenden Substanz, der Dicke des Materials, sowie von dem Partialdruckunterschied der permeierenden Substanz zwischen den beiden Seiten des Materials abhängig.

Die Prüflecks können insbesondere bei allen Leckagemesssystemen eingesetzt werden, mit denen Gegenstände geprüft werden, die Flüssigkeiten beinhalten. Die jeweilige Flüssigkeit oder bei einem Gemisch zumindest eine Komponente des flüssigen Gemischs muss hierzu unter den gewählten Testbedingungen einen Dampfdruck oberhalb von 1 Pa aufweisen, bei Nachweis kleiner Leckagestellen oberhalb von 100 Pa. Bevorzugt weist die Flüssigkeit den für die Messung gewünschten Dampfdruck bereits bei 25°C und umgebendem Atmosphärendruck auf, andererseits muss die Testkammer des Leckagesystems entsprechend hinsichtlich Temperatur und Druck eingestellt werden. Besonders bevorzugt werden die Prüflecks mit der gleichen Flüssigkeit befüllt, die auch in den im Leckagemesssystem zu prüfenden Gegenständen beinhaltet ist. Die zu prüfenden Gegenständen können sehr unterschiedlich sein, insbesondere auch hinsichtlich der Art und Menge der in ihnen eingeschlossenen Flüssigkeit. So ist nicht nur die Dichtigkeitsprüfung von Behältern möglich, die explizit der Flüssigkeitslagerung dienen, sondern auch die Prüfung nahezu beliebiger Gegenstände mit innen eingeschlossener Flüssigkeit. Zu dieser Gruppe von Gegenständen gehören auch solche, in denen die Flüssigkeit indirekt eingeschlossen ist wie beispielsweise bei batteriebetriebenen Geräten, die je nach Anwendung mit besonderer Sorgfalt gegen das Austreten von Batterieflüssigkeit gesichert werden müssen.

Bevorzugt weist das Leckagemessgerät eine Testkammer auf, in die der zu prüfende Gegenstand eingesetzt wird. In der Testkammer sammelt sich während einer definierten bzw. geeigneten Sammelzeit das aus dem Gegenstand austretende Gas. Nach Ablauf der Sammelzeit wird die Luft bzw. das Gasgemisch aus der Testkammer auf Substanzen aus dem Produkt hin analysiert. Dies geschieht vorzugsweise unter Verwendung einer gängigen Analysetechnik wie beispielsweise der Massenspektrometrie, Chromatographie, Flugzeituntersuchungsmethode, Infrarotspektroskopie oder Photoakustik oder unter Verwendung von Gassensoren, insbesondere von Halbleitergassensoren, zu denen das zu analysierende Gasgemisch geleitet wird.

Ein Fokus der Erfindung liegt auf der Eignungsprüfung und Kalibrierung von Leckagemesssystemen für Dichtigkeitsprüfungen an Behältern, die mit flüssigen medizinischen Formulierungen befüllt sind. Ein weiterer Fokus richtet sich auf die Kalibrierung von Dichtigkeitsprüfungen an ganzen Geräten, bevorzugt medizinischen Handgeräten wie Zerstäuber oder Injektoren, mit denen Flüssigkeiten in definierten Volumina ausgebracht, d.h. zerstäubt oder eingespritzt werden.

Der Begriff "Flüssigkeit" umfasst neben reinen Flüssigkeiten und Lösungen zusätzlich Dispersionen, Suspensionen, Suslutionen (Mischungen aus Lösungen und Suspensionen) oder dergleichen. Unter dem Begriff "medizinische Formulierung" oder "Arzneimittelformulierung" sind bei der vorliegenden Erfindung über Medikamente hinaus auch Therapeutica oder dergleichen, insbesondere also jede Art von Mitteln zur Inhalation oder sonstigen Anwendung zu verstehen.

Die einzelnen Merkmale der vorliegenden Erfindung können unabhängig voneinander genutzt oder miteinander kombiniert werden.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
- Fig. 1: eine schematische Darstellung eines Leckagemesssystems, in dem das erfindungsgemäße Prüfleck eingesetzt werden kann,
- Fig. 2: eine schematische Schnittdarstellung eines erfindungsgemäßen Prüflecks,
- Fig. 3: Diagramm bezüglich des Zusammenhangs zwischen Verdunstungsraten von Prüflecks und den ihnen zugehörigen Permeationsflächen, wobei die gravimetrisch bestimmte Leckagerate in Milligramm pro Tag über der Fläche in Quadratmillimeter aufgetragen ist,
- Fig. 4: Diagramm bezüglich des Zusammenhangs zwischen der durch Prüflecks erzeugten Gaskonzentration in der Testkammer und dem am Sensor erzeugten elektrischen Signal, wobei die relative Signalamplitude in Volt über der Konzentration in der Testkammer in der Einheit ppm aufgetragen ist,
- Fig. 5: eine schematische Schnittdarstellung eines im Leckagemesssystem zu prüfenden medizinischen Behälters,
- Fig. 6: eine schematische Schnittdarstellung eines Zerstäubers im ungespannten Zustand mit dem Behälter nach Fig. 5
- Fig. 7: eine schematische Schnittdarstellung des Zerstäubers im gespannten Zustand.

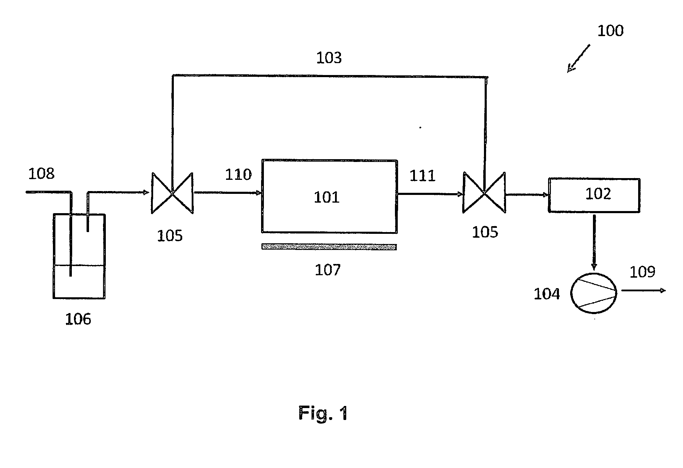

Fig. 1 zeigt den schematischen Aufbau eines exemplarischen Leckagemesssystems (100), in dem ein erfindungsgemäßes Prüfleck (300) in eine Testkammer (101) eingesetzt werden kann. Die Testkammer (101) weist eine dicht verschließbare Öffnung auf, durch welche der zu prüfende Gegenstand eingesetzt werden kann. Desweiteren weist sie einen Einlass (110) für ein Trägergas und einen Gasauslass (111) auf, die unabhängig voneinander über Ventile, vorzugsweise Dreiwegeventile (105) verschlossen werden können. Wahlweise (in Figur 1 nicht dargestellt) kann die Testkammer noch zusätzlich mit dem Anschluss an eine Vakuumpumpe ausgestattet sein, die bei Bedarf die Testkammer (101), in die der zu prüfendem Gegenstand eingesetzt ist, zu Beginn eines Messzyklus evakuiert. Als Trägergas kann ein beliebiges Gas unter beliebigem Druck verwendet werden. Im bevorzugten Beispiel handelt es sich bei dem Trägergas um Umgebungsluft, die über einen Lufteinlass (108) in einer Waschflasche (106) konstant angefeuchtet wird oder mittels anderer Hilfsmittel konditioniert wird, bevor sie über ein Dreiwegeventil (105) in die Testkammer (101) geleitet wird. Die Waschflasche (106) ist vorzugsweise mit entmineralisiertem Wasser gefüllt und gibt der als Trägergas dienenden Luft eine relative Luftfeuchte von über 70%. Dadurch werden etwaige Verunreinigungen wie beispielsweise Partikel oder Lösemitteldämpfen aus der Umgebungsluft entfernt, so dass das spätere Messergebnis durch solche Verunreinigungen nicht verfälscht wird. Mittels der beiden Dreiwegeventile (105) kann eingestellt werden, ob das Trägergas in die Testkammer (101), durch diese hindurch oder durch einen Bypass (103) zum Sensor (102) des Messsystems geleitet wird. Die Strömung des Trägergases im System, vom Ansaugen von Umgebungsluft bis hin zum Transport des Gasgemisches aus der Testkammer (101) zum Sensor (102), wird durch eine Pumpe (104) bewirkt, der wiederum ein Luftauslass (109) zugeordnet ist, der das Zustandekommen einer Strömung ermöglicht. Bei der Pumpe handelt es sich beispielsweise um eine Membranpumpe, die im System Durchflussströmungen von 0,5 bis 2 Liter pro Minute, vorzugsweise 1 bis 1,5 Liter pro Minute erzeugt. Der Durchfluss, beispielsweise mit einem kalibrierten thermischen Massendurchflussmesser ermittelt, kann je nach Ventilstellung oder Messphase variieren. Bedingt durch den Sog, den die Pumpe erzeugt, kann in den Trägergasleitungen und der Testkammer (101) zuweilen ein geringfügiger Unterdruck gebildet werden.
Zur Messung wird der zu prüfende Gegenstand in die Testkammer (101) eingesetzt, die vorzugsweise aus Metall ist und fest verschlossen wird. Der Ablauf einer Messung ist beispielsweise wie folgt: Der Messablauf startet mit einer Vorspülzeit, in der die Dreiwegeventile (105), so geschaltet werden, dass eventuelle Verunreinigungen und insbesondere solche, die dem Gegenstand von außen anhaften, aus der Testkammer entfernt werden. Nach Ablauf der Vorspülzeit werden die Ventile zum Bypass (103) geschaltet, so dass der Trägergasstrom außerhalb der Testkammer (101) direkt zum Sensor (102) umgeleitet wird. Somit erzeugt dieses über den Bypass (103) kommende Trägergas am Sensor ein Leerwert-Signal oder die so genannte Basislinie, d.h. das Signal, das ohne die Gegenwart von zu detektierenden Bestandteilen am Sensor anliegt. Mit der Umstellung der Ventile auf die Strömung des Trägergases durch den Bypass (103), d.h. mit dem Verschließen von Einlass (110) und Gasauslass (111) der Testkammer (101), beginnt die Sammelzeit in der Testkammer (101). In dieser Zeit sammelt sich das aus dem Gegenstand austretende und im Leckagemesssystem nachzuweisende Gas oder der nachzuweisende Dampf in der Luft innerhalb der Testkammer (101). Bei der hier dargestellten Ausführungsform herrscht in der Testkammer (101) während der Sammelzeit Normaldruck oder ein geringfügiger Unterdruck. Für den Austritt des Gases oder Dampfes oder der flüchtigen Flüssigkeitsbestandteile aus dem Gegenstand sind neben der Art des stattfindenden Leckageprozesses die Partialdruckdifferenz zwischen dem Innen- und Außenraum des Gegenstandes sowie der in ihm herrschenden Innendruck maßgebend.
Mittels einer durch die Markierung 107 in Figur 1 angedeuteten Heizung lässt sich die Testkammer (101) optional auf eine gewünschte Temperatur oberhalb der Raumtemperatur aufheizen. Durch dieses Aufheizen kann das Austreten des zu detektierenden Gases aus dem Gegenstand beschleunigt werden. Die Heiztemperatur, optional einstellbar zwischen Raumtemperatur und 150°C, sollte so gewählt werden, dass sie unterhalb der Siedetemperatur der im Gegenstand vorhandenen Flüssigkeit ist, damit das resultierende Messergebnis nach wie vor auf Leckageprozessen basiert, wie sie bei Raumtemperatur vorliegen. Ebenfalls sollte eine solche Heiztemperatur unterhalb jeglicher Materialverformungstemperaturen des Gegenstandes liegen. Vorzugsweise liegt die Temperatur in der Testkammer (101) im Bereich von 20°C bis 60°C. In einer Ausführungsvariante kann die Heizung (107) auch zusätzlich ein Vorheizen sowohl des zu prüfenden Gegenstandes und/oder des Trägergases auf die in der Testkammer (101) herrschende Temperatur bewirken.
Nach Abschluss der Sammelzeit werden die Ventile zur Injektion geschaltet; die um das aus dem Gegenstand ausgetretene Gas angereicherte Luft wird nun zum Sensor geleitet. Nach Abschluss der Injektionszeit werden die Ventile erneut auf die Strömung entlang des Bypasses (103) geschaltet und die Nachlaufzeit beginnt. Diese dient der Spülung des Sensors und/oder der Ermittlung einer weiteren Basislinie. Mit Hilfe beider Basislinien kann im Anschluss die Auswertung des Sensorsignals beispielsweise über eine Peakerkennung mit Peakflächenintegration erfolgen.
Vorzugsweise ist das Leckagemesssystem (100) softwaregesteuert, d.h. Ventilstellungen, Durchfluss der Pumpe (104), Temperatur der Testkammer (101) und des Sensors (102), Vorspül- und Nachlaufzeit, Dauer der Sammelzeit und der Inj ektionsphase sowie die Anzahl an Injektionen werden entsprechend von Dateneingaben über ein Computerprogramm gesteuert.
In einer bevorzugten Ausführungsform ist der Sensor (102) ein Halbleitergassensor, der besonders empfindlich für Dämpfe von organischen Verbindungen ist. Der unmittelbar auf die Dämpfe reagierende Sensor (102) erzeugt einen von der Gaskonzentration abhängigen Messwert. Ein solcher Sensor (102) kann innerhalb von Sekundenbruchteilen auf Konzentrationsänderungen ansprechen.
Die Funktion des hier eingesetzten Halbleitergassensors basiert bevorzugt auf Chemisorptionsprozessen oder chemischen Reaktionen an einer halbleitenden Metalloxidschicht. Bevorzugt beinhaltet der Sensor zwei Schichten, zum einen eine Heizerschicht und zum anderen eine sensitive Schicht. Die an einen Stromkreislauf angeschlossene Heizerschicht heizt die darüber liegende sensitive Schicht. Die Temperatur des Sensors (102) wird isotherm erhöht gehalten, vorzugsweise bei 200°C bis 600°C, besonders bevorzugt bei 350°C bis 450°C insbesondere bei 400°C. In der sensitiven Schicht erfolgt eine Änderung der elektrischen Leitfähigkeit in Abhängigkeit von der Art und Konzentration des am Sensor (102) vorliegenden Gases. Für die sensitive Schicht des Halbleitergassensors werden beispielsweise SnO₂ (Zinndioxid), In₂O₃ (Indiumoxid), WO₃(Wolframoxid), Ga₂O₃ (Galliumoxid) oder ZnO (Zinkoxid) verwendet. Durch Sauerstofffehlstellen oder Störungen in der Kristallstruktur ist die Erzeugung von Ladungsträgern in der halbleitenden Schicht möglich. Dieser Effekt ist temperaturabhängig; die Leitfähigkeit steigt mit einer Erhöhung der Temperatur. An der Oberfläche werden reversibel Verbindungen mit Luftsauerstoff eingegangen, wobei freie Ladungsträger des Metalloxids gebunden werden und die Leitfähigkeit erniedrigt wird. Kommt dagegen statt des oxidierenden Sauerstoffs ein reduzierendes Gas an die Sensoroberfläche, so reagiert dieses mit dem aus der Luft adsorbierten Sauerstoff, Ladungsträger werden frei und die Leitfähigkeit steigt. Da die Widerstandsänderung der Sensorschicht im Prinzip durch alle reduzierenden Gase hervorgerufen wird, weist ein solcher Halbleitergassensor eine geringe Selektivität auf. Allerdings können unterschiedliche gasspezifische Aktivierungsenergien durch unterschiedlich hohe Sensorschichttemperaturen ausgenutzt werden. Auf diese Weise kann bei einer niedrigeren Betriebstemperatur ein Gas am Sensor (102) erkannt werden, während ein anderes erst bei höheren Temperaturen erkannt wird. Auf diese Weise lässt sich zumindestens eine partielle Selektion am Sensor (102) einstellen. Generell sind bei Verwendung von Halbleitergassensoren alle potentiellen Störquellen bei so einer Messung zu vermeiden. Beispielsweise werden beim Aufbau des Leckagemesssystems nur ausgasungsfreie Materialien verwendet. Alternativ kann anstelle eines einzelnen Sensors (102) auch ein selektives Messgerät wie beispielsweise ein Massenspektrometer, ein Gaschromatograph, ein Flugzeitspektrometer, ein photoakustisches Messsystem oder ähnliches an das Ventil hinter der Testkammer (101) angeschlossen werden. Der Anschluss eines solchen Messgeräts hat den Vorteil, dass auch verschiedene aus dem zu prüfenden Gegenstand austretende Gase gleichzeitig selektiv erkannt werden können.

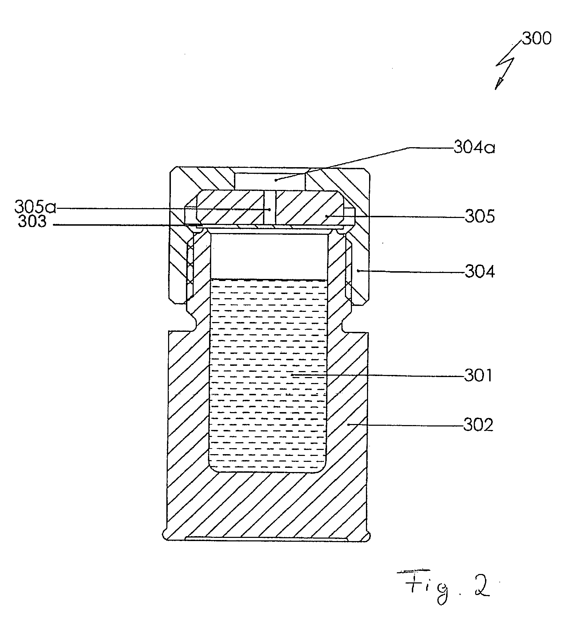

Fig. 2 zeigt eine schematische Schnittdarstellung durch eine Ausführungsform eines erfindungsgemäßen Prüflecks (300). Das Prüfleck (300) umfasst ein starres Gefäß (302), einen Deckel (304), eine optionale Stützscheibe (305), eine Membran oder Folie (303) und Flüssigkeit im Inneren des Prüflecks. Die Flüssigkeit ist vorzugsweise ein Lösemittel wie beispielsweise Ethanol. Im Ausführungsbeispiel verschließt der Deckel (304) das Gefäß (302) dicht, wobei Stützscheibe (305) und Membran oder Folie (303) innen festgeklemmt sind. Bevorzugt bildet die Membran oder Folie (303) eine seitliche Abdichtung des Prüflecks. Vorzugsweise ist die Membran oder Folie (303) zwischen dem Rand des Gefäßes (302) und der Stützscheibe (305) eingeklemmt. Deckel (304) und Stützscheibe (305) sind hierbei vorzugsweise verformungsstabil. Die Stützscheibe (305) und der Deckel (304) weisen Bohrungen (305a) und (304a) auf, durch die an der vom Gefäß (302) abgewandten Oberfläche der Folie (303) ein Luftaustausch mit der Umgebung stattfinden kann. Bevorzugt ist die Membran oder Folie (303) elastischer oder oberflächenweicher als der Rand des Gefäßes (302) und die Stützscheibe (305), so dass sie insbesondere durch die Verpressung zwischen Rand des Gefäßes (302) und Stützscheibe (305) die Dichtung vom Innenraum des Prüflecks nach außen bildet. Optional können Stützscheibe (305), Folie (303) und Deckel (304) als dreiteilige Verschlusskappe des Prüflecks (300) ausgebildet sein. Alternativ (nicht dargestellt) können die Funktionen von Deckel (304) und Stützscheibe (305) auch von einem einzigen Bauteil beispielsweise von einem spritzgusstechnisch hergestellten Deckel (304) übernommen werden, der mit einer von oben nach innen ragenden Kante oder Kontur die Membran oder Folie direkt mit dem oberen Rand des Gefäßes verpresst und eine innenliegende Nut aufweist, in die das Material der Membran oder Folie (303) hineinragen kann, das über den oberen Rand des Gefäßes (302) übersteht. Alternativ (ebenfalls nicht dargestellt) kann die Membran oder Folie (303) in spritzgusstechnischen Verfahren direkt in den Deckel (304) intergriert werden und ein zusätzliches Dichtelement kann die Abdichtung der Verbindung von Gefäß (302) zu Deckel (304) sicherstellen.

Vorzugsweise sind Deckel (304) und Gefäß (302) miteinander verschraubt, so dass man das Prüfleck (300) je nach Anforderung neu verändert aufbauen kann. Diese Variante eignet sich für Laborbetriebe, bei denen flexibel die Eignung von Leckagemesssystemen für die Prüfung unterschiedlicher Gegenstände wie z.B. unterschiedlich befüllter Behälter geprüft werden soll. Bevorzugt wird - wie auch in Figur 2 dargestellt - eine Verschraubung über ein Innengewinde am Deckel (304) und ein Außengewinde am Gefäß (302) hergestellt, oder in einer nicht dargestellten Variante über ein Außengewinde am Deckel (304) und ein Innengewinde am Gefäß (302). Durch das Aufschrauben presst der Deckel (304) die im Beispiel lose Stützscheibe (305) auf die auf dem Rand des Gefäßes (302) aufliegende Folie (303). Dadurch wird die Membran oder Folie (303) ihrerseits zwischen Gefäß (302) und Stützscheibe (305) verpresst. Die Membran oder Folie (303) dichtet so den Innenraum des Prüflecks (300) derart ab, dass durch die Verbindungsstelle zwischen Deckel (304) und Gefäß (302), insbesondere durch den Gewindespalt, keine Permeation oder Diffusion oder kein Gasaustausch mit der äußeren Umgebung stattfinden kann. Optional kann unabhängig von der Art der Verbindung zwischen Deckel (304) und Gefäß (302) eine Dichtung an der Verbindungsstelle von Deckel (304) und Gefäß (302) eingebaut werden. Diese Dichtung kann ein zusätzliches vorzugsweise elastomeres Bauteil umfassen oder alternativ durch die Verpressung eines Bauteils aus einem oberflächenhärteren Material mit einem Bauteil aus einem oberflächenweicheren Material gebildet werden. Alternativ kann auch ein elastomerer oder oberflächenweichere Materialbereich fest an eines der Bauteile wie beispielsweise am Deckel (304) oder am Gefäß (302) beispielsweise mittels spritzgusstechnischer Verfahren angebunden sein.

In einer alternativen Ausführungsform können Deckel (304) und Gefäß (302) auch beispielsweise über eine Crimpverbindung unlösbar mit einander verbunden sein. Solche nicht aufmachbaren Prüflecks eigenen sich für routinemäßig in Produktionsprozesse eingebundene Dichtigkeits- oder Leckagemessungen, bei denen die zu prüfenden Gegenstände kaum variieren und das System immer auf die gleichen Grenzwerte reagieren muss.

Alle angesprochen Varianten weisen eine Flüssigkeit, die sich im Gefäß (302) befindet, und eine Membran oder Folie (303) auf, welche den offenen Bereich des Gefäßes (302) bedeckt. Gase oder Flüssigkeitsbestandteile, die durch die bevorzugt dünne Folie (303) über Permeation oder Diffusion hindurch dringen, können dem Prüfleck durch eine Bohrung (304a) im Deckel (304) ungehindert entweichen. Im Ausführungsbeispiel mit Stützscheibe (305) weist diese ebenfalls eine Bohrung (305a) auf. Im bevorzugten Ausführungsbeispiel haben die Bohrungen (304a, 305a) Durchmesser im Bereich von 1 bis 20 Millimetern einschließlich.
Überraschenderweise hat es sich gezeigt, dass die Gasaustrittsrate des Prüflecks (300) von der den Innenraum des Gefäßes (302) begrenzenden Fläche der Membran oder Folie (303) und nicht von den Querschnitten der nachfolgenden Bohrungen (304a, 305a) abhängen. Das Prüfleck funktioniert gleichermaßen auch bei Vorliegen eines flächigen Spaltes zwischen Membran oder Folie (303) und Stützscheibe (305), sofern eine umlaufende - vorzugsweise von der Membran oder Folie (303) selbst gebildete - Dichtung am äußeren Rand zwischen Membran oder Folie (303) und Stützscheibe (305) besteht.

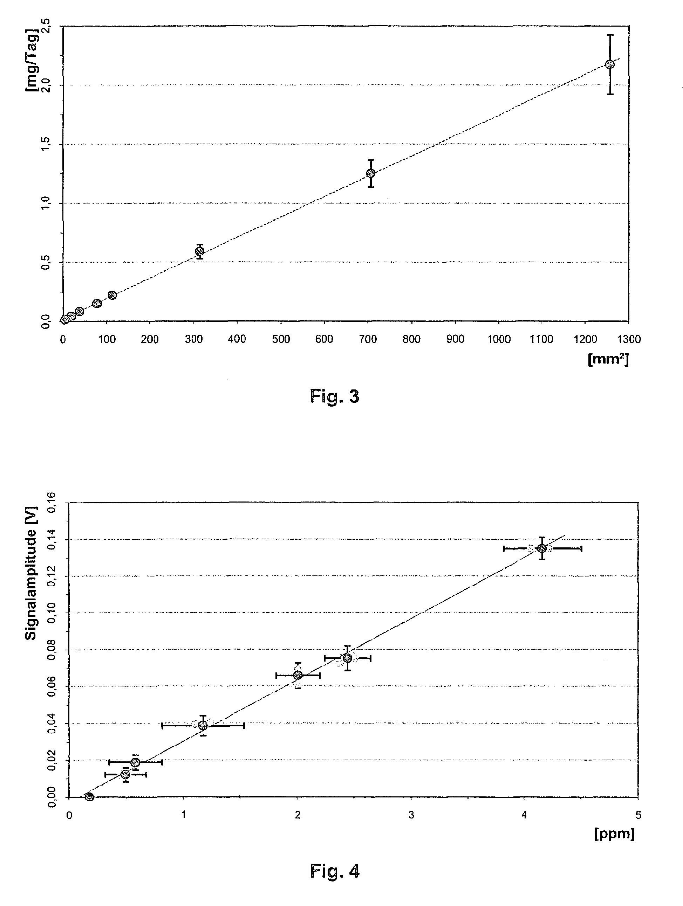

Fig. 3 zeigt eine durch Differenzwiegung bestimmte Abhängigkeit der Leckagerate aus einer Reihe von Prüflecks (300). Die Prüflecks (300) unterscheiden sich hierbei bezüglich der Größe der Folienfläche, durch die das innere Volumen des Gefäßes (302) abgeschlossen wird oder die einen Teil der inneren Gefäßwand bildet und auf deren gegenüberliegenden Seite sich ein mit der Umgebungsluft kommunizierender freier Raum befindet. Im Diagramm ist die Leckagerate in Milligramm pro Tag über der Fläche in Quadratmillimeter aufgetragen. Für die hier abgebildete Messreihe wurden Prüflecks (300) gemäß dem in Fig. 2 dargestellten Aufbau verwendet. Die Prüflecks hatten einen radial-symmetrischen Aufbau, die äußere Form war im Wesentlichen zylindrisch. Das Gefäß (302) und die Stützscheibe (305) waren aus Edelstahl und der Deckel (304) aus PTFE (Polytetrafluorethylen). Die Folie (303) bestand aus einer 100µm dicken PE-Folie (Polyethylen). Die Prüflecks (300) enthielten ein mit Ethanol vollgesogenes Speichermaterial (301) im Inneren des Gefäßes (302). Der über dem Inneren des Gefäßes (302) anliegende Folienquerschnitt wurde zwischen 15 Quadratmillimeter und 1300 Quadratmillimetern variiert und es ergab sich ein reproduzierbarer linearer Zusammenhang zwischen Leckagerate und über dem Inneren des Gefäßes (302) anliegende Oberfläche der Folie (303). Der direkt am Innenraum des Gefäßes (302) anliegende Querschnitt bzw. die hier betrachtete Oberfläche der Folie (303) entspricht dem in diesem radial symmetrischen Ausführungsbeispiel kreisförmigen Flächenbereich, der außen durch die ringförmig umlaufende Verpressung der Folie zwischen oberem Rand des Gefäßes (302) und gegenüberliegender Stützscheibe (305) begrenzt wird. Die Leckagerate oder Verdunstungsrate der Prüflecks (300) wurde für die der Figur 3 zugrunde liegenden Messwerte gravimetrisch bestimmt. Zur Erzeugung dieser Messwerte wurden die einzelnen Prüflecks (300) über eine Zeitspanne von 6 Wochen regelmäßig auf einer Analysenwaage gewogen und der durch die Leckage bzw. Verdunstung der Flüssigkeit hervorgerufene Gewichtsverlust ermittelt. In gleicher Regelmäßigkeit wurden in einem Leckagemesssystem gemäß Figur 1 die Signale ermittelt, die durch die Verdunstungsraten derselben Prüflecks (300) in einem Leckagemesssystem (100) in Figur 1 entstanden. In Figur 4 ist die Amplitude der elektrischen Sensorsignale in Volt über der Ethanolkonzentration in der Testkammer in der Einheit ppm aufgetragen. Das durch den Sensor (102) erzeugte elektrische Signal hängt, wie diese in Figur 4 abgebildeten Messungen bestätigten, linear von der in der Testkammer (101) vorliegenden Gaskonzentration ab. Somit hängt das Signal zugleich auch linear von der ursächlichen Verdunstungsrate des in das Leckagemesssystem (100) eingesetzten zu prüfenden Gegenstands oder wie hier des eingesetzten Prüflecks (300) ab. Sowohl in Figur 3 als auch in Figur 4 entsprechen die Fehlerbalken, die von den als Messpunkten eingetragenen Mittelwerten ausgehen, den Standardabweichungen der jeweiligen Messwerteschar bei Wiederholungsmessungen bzw. über die Dauer der Messungen.

Das Figur 3 und Figur 4 zugrundeliegende Beispiel verdeutlicht das Prinzip des erfindungsgemäßen Verfahrens zur Überprüfung oder Kalibrierung von mit Testkammern (101) ausgestatteten Leckagemesssystemen (100) mittels Prüflecks (300): Falls noch nicht bekannt, werden zunächst die äußeren Volumina von Prüfleck (300) und zu prüfendem Gegenstand und das innere Volumen der Testkammer (100) bestimmt. Ein Prüfleck (300) mit bekannter Verdunstungs- oder Leckagerate - beispielsweise gravimetrisch oder auf Basis der Permeationsprinzipien rechnerisch bestimmt - wird in die Testkammer (101) eingesetzt. Die am Ende der vordefinierten Sammelzeit in der Testkammer (101) vorliegende Konzentration berechnet sich aus der Menge der aus dem Prüfleck (300) ausgetretenen Substanz und dem Differenzvolumen von Prüfleck (300) und Testkammer (101). Am Sensor wird ein Signal entsprechend der Gaskonzentration über der Basislinie erzeugt, dessen Amplitude nun direkt der bekannten Verdunstungsrate am Prüfleck (300) zugeordnet werden kann. Durch die Messung von verschiedenen Prüflecks (300) mit unterschiedlichen Verdunstungsraten kann auf diese Weise auch eine nicht-lineare Messsignal-Erzeugung am Sensor überprüft werden. Auch ein nicht-linear auf die Gaskonzentration in der Testkammer (101) reagierender Sensor kann so mittels eines durch Prüflecks (300) bestimmten Umrechnungsschemas oder anhand von Prüflecks (300) überprüften mathematischen Funktionszusammenhangs zum Einsatz gebracht werden. Die bei der Überprüfung von Leckagemesssystemen (100) eingesetzten Prüflecks (300) befinden sich zum Zeitpunkt ihrer Verwendung bevorzugt im so genannten stationären Zustand. In diesem stationären Zustand hat sich eine konstante Verdunstung oder Leckage oder Permeation eingestellt, d.h. die zugehörige Verdunstungs- oder Leckagerate ist konstant. Wird ein Prüfleck (300) mit einer zur Verdunstung führenden Flüssigkeit befüllt, so weist es erst dann eine konstante Verdunstungsrate auf, wenn einerseits alle dem Prüfleck (300) von außen anhaftenden, verdunstende Flüssigkeitsreste verdunstet sind und sich andererseits an der relevanten Verdunstungsbarriere des Prüflecks ein Gleichgewichtszustand bezüglich der beteiligten Permeations- und Diffusionsprozesse ausgebildet hat. Die Zeitdauer bis zum Erreichen des stationären Gleichgewichtszustands hängt vom Diffusionskoeffizienten des durch die Verdunstungsbarriere permeierenden Gases, von der Struktur, Oberfläche und vor allem Dicke der als Verdunstungsbarriere im Prüfleck (300) eingesetzten Membran oder Folie (303) und von den klimatischen Bedingungen wie insbesondere Temperatur und äußeren partiellen Gasdrücken ab. Vorzugsweise liegen zwischen Befüllung bzw. Aufbau eines Prüflecks (300) und seiner Verwendung 1 bis 24 Stunden, insbesondere mehr als 4 und ganz besonders bevorzugt mehr als 16 Stunden.

Besonders bevorzugt wird die Verdunstungsrate des Prüflecks (300) durch Permeation durch eine Membran oder Folie (303) bestimmt. Als Permeation wird der Teilchentransport durch einen Festkörper bezeichnet, welcher in vier aufeinanderfolgende Teilprozesse untergliedert werden kann: Adsorption der Atome oder Moleküle an der Membran- oder Folienoberfläche aus einem anliegenden Gas oder einer anliegenden Flüssigkeit, Absorption der Atome oder Moleküle in die Membran oder Folie (303), Diffusion insbesondere quer durch die Membran oder Folie (303) entlang eines Konzentrationsgradienten und Desorption von der anderen bzw. gegenüberliegenden Oberfläche der Membran oder Folie (303) in den dort anliegenden bevorzugt freien bzw. gasgefüllten Raum. Besonders bevorzugt wird als Membran oder Folie (303) ein Polymermaterial eingesetzt. Bei Polymeren laufen in der Regel die sowohl die Adsorption als auch die Desorption im Vergleich zur Diffusion sehr viel schneller ab. Die Diffusion ist daher die zeitlich bestimmende Größe für den Stofftransport durch das Polymer, d.h. für die Permeation am Prüfleck (300) im hier betrachteten Fall.

Besteht der die Verdunstungsbarriere bildende Festkörper aus einem homogenen Polymer, so kann die Permeation eines Gases - eine für das Prüfleck (300) bevorzugte Situation - durch das so genannte Lösungs-Diffusions-Modell beschrieben werden. Danach folgt die Permeation einem Konzentrations- oder Druckgradienten durch die Polymermatrix. Das Lösungs-Diffusions-Modell gilt ausschließlich für die Permeation eines Gases durch einen Festkörper, bestehend aus einer homogenen Polymerschicht im stationären Zustand, in dem die Konzentrationsverhältnisse zeitlich nicht variieren und die Material- und Diffusionseigenschaften überall im Polymer identisch sind.

Mathematische Betrachtungen können auf Basis des 1. Fick'schen Gesetz zur Diffusion und des Henry'schen Gesetz zur Sorption durchgeführt werden (nachzulesen in "The Mathematics of Diffusion" 2. Edition, 1975, Clarendon Press von J. Crank). Das 1. Fick'sche Gesetz beschreibt den eindimensionalen Gasdurchgang durch eine Probefläche im stationären Zustand, wobei die Menge der transportierten Substanz in der Zeit durch die Fläche diffundiert. Bei solchen Betrachtungen werden für gewöhnlich die in die Berechnung eingehenden Diffusionskoeffizienten und Löslichkeitskoeffizienten bei den üblichen Messbedingungen als konzentrations- bzw. druckunabhängig behandelt. Der Diffusionskoeffizient ist das Maß für die Beweglichkeit der Teilchen und er ist das Maß für die Geschwindigkeit, mit der das Gas durch die Polymermatrix tritt. Der Löslichkeitskoeffizient beschreibt die Löslichkeit des Gases im Polymer. Der Permeationskoeffizient als Maß für den Teilchenstrom durch das Polymer ist das Produkt aus dem Diffusionskoeffizienten und dem Löslichkeitskoeffizienten der permeierenden Substanz. Die Diffusions- und Löslichkeitskoeffizienten sind stark temperaturabhängig - die Abhängigkeit kann mit der in der Wissenschaft bekannten Arrhenius Gleichung abgebildet werden. Mit steigender Temperatur wird die Diffusion für Gase und Flüssigkeiten beschleunigt, d.h. der Diffusionskoeffizient wird größer. Dagegen wird die Löslichkeit mit steigender Temperatur nur bei Gasen erhöht. Für Flüssigkeiten und Dämpfe wird der Löslichkeitskoeffizient mit steigender Temperatur kleiner. Mit steigender Temperatur tendieren die Molekülsegmente des Polymers zu stärkeren Bewegungen, mit der Folge, dass ein diffundierendes Molekül zwischen den inter-und intramolekularen Bereichen des Polymers leichter durchdringen wird.
Aus diesem Grund wird das Prüfleck (300) bevorzugt bei konstanten Temperaturen eingesetzt, so dass bei gravimetrischer Kontrolle des Prüflecks (300) die gleichen Verdunstungsraten vorliegen wie beim Einsatz im Leckagemesssystem (100). Die "normale" Temperatur für den Einsatz eines Prüflecks (300) liegt somit in der Regel bei einer Raumtemperatur von 20°C oder 25°C oder einem dazwischen liegenden Wert. Wenn bei der Messung eines zu prüfenden Gegenstandes und somit auch die bei der Vergleichsmessung mit einem Prüfleck (300) bei einer gegenüber der normalen Temperatur erhöhten Temperatur eingesetzt wird, erhöht sich die Verdunstungsrate des Prüflecks (300). Die exakte Höhe der Verdunstungsrate kann mit Hilfe der angegebenen Gesetzmäßigkeiten für die jeweilige Temperatur errechnet werden.

Gemäß einer weiteren (nicht dargestellten) Ausführungsform kann das Prüfleck (300) selbst eine Heizung oder Temperaturregelung aufweisen, durch die Flüssigkeit und/oder Verdunstungsbarriere und/oder Gasräume unterhalb und/oder oberhalb der Verdunstungsbarriere auf einer konstanten Temperatur gehalten werden können. Die Verdunstungs- oder Leckagerate eines Prüflecks (300) mit so einer zusätzlichen Ausstattung ist weitgehend unabhängig von der Umgebungstemperatur oder dem im Leckagemesssystem (100) vorliegenden Temperaturen, insbesondere sofern die innerhalb des Prüflecks eingestellte Temperatur höher als die Umgebungstemperatur ist. Mit Hilfe einer solchen Temperaturerhöhung können Prüflecks (300) mit konstant erhöhten Verdunstungs-oder Leckageraten aufgebaut werden. Weist das Prüfleck eine Temperaturregelung und möglicherweise zusätzlich auch eine Temperaturanzeige auf, so kann ein einziges insbesondere im Inneren temperiertes Prüfleck beispielsweise bei der Kalibrierung eines Leckagemesssystems (100) anstelle der vormals beschriebenen Gruppe verschieden verdunstender Prüflecks zur Nachstellung unterschiedlicher Gaskonzentrationen in der Testkammer (101) verwendet werden.

Vor dem Hintergrund des Permeationsprozesses weist die in das Prüfleck (300) eingebaute Verdunstungsbarriere ein Material und eine Dicke auf, durch welche die Permeation des Gases oder der flüchtigen Bestandteile aus dem Inneren des Prüflecks (300) möglich ist. Die Auswahl des Materials und seiner Dicke wird durch das jeweilige abzugebende Gas oder der im Inneren des Prüflecks (300) befindlichen Flüssigkeit bestimmt. Vorzugsweise besteht die Festkörperschicht, welche die Verdunstungsbarriere darstellt, aus einer Membran, wie beispielsweise einem Filtermaterial oder einem aus komprimierten oder vernetzten Fasern aufgebauten Material, oder aus einer Kunststofffolie, vorzugsweise aus einem Polymer. Solche Polymere sind beispielsweise PP (Polypropylen), Polyester wie PET (Polyethylenterephthalat), PVC (Polyvinylchlorid), PVDC (Polyvinylidenchlorid), PCTFE (Polychlorotrifluoroethylen), PTFE (Polytetrafluorethylen) und insbesondere die für ethanolische Prüflecks (300) bevorzugten Materialen PE (Polyethylen), Polystyrol und Acetal. Auch der Einsatz von Folien aus verschiedenartigen Schichten oder aus mehreren Komponenten wie beispielsweise PVC-PVDC-PVC- Folien oder PVC-PVDC-Folien oder gar metallisierten oder Metallschichten enthaltenden Folien ist möglich, insbesondere falls besonders geringe oder besonders selektive Permeationen am Prüfleck (303) eingestellt werden sollen. Ebenfalls möglich ist der Einsatz von Membranen mit funktionalisierten Oberflächen. Mittels so einer Funktionalisierung beispielsweise in Form eines Anionen- oder Kationenaustauschs kann die Permeation, insbesondere die Adsortion und Desorption, bestimmter Atome oder Moleküle erhöht oder erniedrigt werden.

Die im Prüfleck (300) verwendete Membran oder Folie (303) hat vorzugsweise eine Dicke im Bereich von wenigen Mikrometern, beispielsweise 10 Mikrometern, bis zu einem Millimeter, bevorzugt eine Dicke zwischen 20 und 500 Mikrometern und ganz besonders bevorzugt von 50 oder 100 Mikrometern oder einem dazwischen liegenden Wert. So lange die Folie (303) der gewählten Dicke generell die Permeation des gewünschten Gases zulässt, bestimmt die Dicke der Folie (303) lediglich die Zeit, die das Prüfleck (300) bis zum Erreichen seines stationären Zustands benötigt. Anschließend weisen Prüflecks (300), die sich lediglich bezüglich der Dicke der eingebauten Folie (303) unterscheiden, die gleichen Verdunstungs- oder Leckageraten auf. Bei dünneren Folien erfolgt die Gleichgewichtseinstellung des Konzentrationsgradienten von der inneren zur äußeren Oberfläche der Folie (303) schneller.

Für den Einsatz in einem erfindungsgemäßen Prüfleck (303) sind alle permeierende Lösemittel geeignet. Solche Lösemittel sind beispielsweise
- Alkohole (Methanol, Propanol, Isopropanol, Butanol etc.)
- Ketone (Aceton, Butanon etc.)
- Ester (Ethylacetat, Butylester etc.)
- Aldehyde (Formaldehyd in Lösung (Formalin), Acetaldehyd, Propionaldehyd etc.)
- Alkane (Hexan, Heptan, Oktan etc.)
- Kohlenwasserstoffe (Benzol, Toluol, Xylol, Chloroform, Dichlormethan, Tetrachlorethylen etc.)
- Nitrile (Acetonitril, Propionitril, Butyronitril etc.)
- Lactone (4-Butyrolacton etc.)
- Sulfoxide (Dimethylsulfoxid etc.)
- Terpene / Öle (Limonenöl, Pfefferminzöl, Orangenöl, Campheröl, etc.).

Das Prüfleck (303) ist darüber hinaus bei allen Flüssigkeiten oder Lösemitteln, wie z.B. die unpolaren Lösemittel (Alkane, Alkene, Alkine, Benzole, Aromate, Ether, Silane, Sulfide usw.) und polaren Lösemittel (Ketone, Ester, Lactone, Nitrile, Amide, Sulfoxide, Sulfone usw.), sowie der polaren Lösemittel (Wasser, Alkohole, Carbonsäuren, Amine, Mineralsäuren usw.) einsetzbar.

Die Flüssigkeit im Prüfleck (300) kann sowohl in ein Speichermaterial oder eine Matrix eingebunden oder als so zu sagen "lose" Flüssigkeit im Inneren des Gefäßes (302) vorliegen. Die Markierung 301 in Figur 2 kann sich auf beide Zustände beziehen, im Folgenden wird sie für das vollgesogene Speichermaterial verwendet. Bevorzugt befindet sich im Inneren des Gefäßes (302) ein mit der Flüssigkeit vollgesogenes Speichermaterial (301). Dieses Speichermaterial (301) nimmt die Flüssigkeit derart auf, dass es selbst von ihr nass oder durchnässt ist und von dieser nassen Oberfläche die Flüssigkeit verdunsten kann. Vorzugsweise ist gerade so viel Flüssigkeit im Inneren des Gefäßes (302), dass sie komplett vom Speichermaterial aufgesogen wurde und keine "lose" Flüssigkeit im Prüfleck (300) in Bewegung ist. Auf diese Weise kann sichergestellt werden, dass es zu keinen Flüssigkeitsleckagen am Prüfleck (300) kommt, sondern Flüssigkeit nur gasförmig und vorzugsweise nur durch Permeations- und Diffusionsprozesse aus dem Prüfleck (300) austritt. Das Speichermaterial (301) hat vorzugsweise eine sehr große Oberfläche und/oder eine sehr große Porosität. Es kann die Form einer Matrix oder einer Tamponade haben. Beispielsweise kann das Speichermaterial (301) durch einen Schwamm, ein Flies, Zellulosematerial wie Watte oder Filtermaterial gebildet werden. Vorzugsweise ist das Speichermaterial (301) so im Prüfleck (300) eingesetzt, dass zwischen seiner Oberfläche und der unteren Oberfläche der Membran oder Folie (303) ein kleiner Hohlraum besteht, in dem sich eine Sättigung mit dem aus der Flüssigkeit verdunstenden Gas einstellen kann. Dies unterstützt die Ausbildung eines unveränderlichen Konzentrationsgradienten in der Membran oder Folie (303), so dass die Konstanz der Verdunstung- oder Leckagerate des Prüflecks (300) sichergestellt ist.

Für beide Ausführungsbeispiele gilt, dass das Prüfleck (300) eine konstante Verdunstungsrate aufweist, so lange der Konzentrationsgradient des aus der Flüssigkeit stammenden Gases in der Folie unverändert ist. Für das Ausführungsbeispiel mit Speichermaterial (301) bedeutet dies, dass die Verdunstung des Prüflecks (300) so lange konstant sein kann wie sich Flüssigkeit im Speichermaterial (301) befindet. Im Falle von getesteten Prüflecks (300) konnten beispielsweise bei vergleichsweise geringer Befüllung in der Größenordnung von 1 Milliliter Ethanol konstante Verdunstungsraten von über einem Jahr erzielt werden.

Das Prüfleck (300) ähnelt in seinen äußeren Abmessungen, insbesondere in Durchmesser und Höhe bevorzugt dem im jeweiligen Leckagemesssystem zu prüfenden Gegenstand. Insbesondere entspricht sein äußeres Gesamtvolumen dem des zu prüfenden Gegenstandes. Dies hat den Vorteil, dass sich nach Einsatz des Prüflecks in die Testkammer (101) und nach Verschließen der Testkammer (101) die gleiche Menge Luft oder Trägergas in der Testkammer (101) befindet, wie bei der Messsituation des zu prüfenden Gegenstandes. Aus dem Prüfleck entweichende Gasmengen oder Substanzmengen erzeugen also die gleiche Konzentration im Gasgemisch der Testkammer (101) wie identische Gasmengen oder Substanzmengen, die aus dem zu prüfenden Gegenstand entweichen können. Weicht die Größe des Prüflecks (300) von der Größe des zu prüfenden Gegenstandes ab, so resultiert die entsprechende Volumenabweichung in einer Änderung der Gaskonzentrationen bei gleicher aus dem zu prüfenden Gegenstand austretenden Gas- oder Substanzmenge. Die Auswertung der Signale am Sensor (102) oder der Messwerte am angeschlossenen Messsystem muss dann also um diese Konzentrationsabweichung gegenüber dem Prüfleck (300) korrigiert werden. Im hier gewählten Darstellungsbeispiel in Fig. 2 wird ein Prüfleck (300) dargestellt, der zur Kalibrierung oder Eignungsprüfung bei Dichtigkeitsprüfungen von mit Flüssigkeit befüllten Behältern (3) eingesetzt wird. Vorzugsweise hat ein solcher Behälter (3) die Gestalt einer Kartusche oder Phiole. Gemäß generell gültigen Forderungen an in Leckagemesssystemen verwendeten Komponenten, bestehen die Einzelteile des Prüflecks (300) aus ausgasungsarmen Materialen, vorzugsweise Metall wie beispielsweise Edelstahl, Aluminium oder Messing oder aus geeigneten vorzugsweise chemisch resistenten Kunststoffen wie beispielsweise PTFE oder aus Glas (mögliche Materialien für das Gefäß (302)).

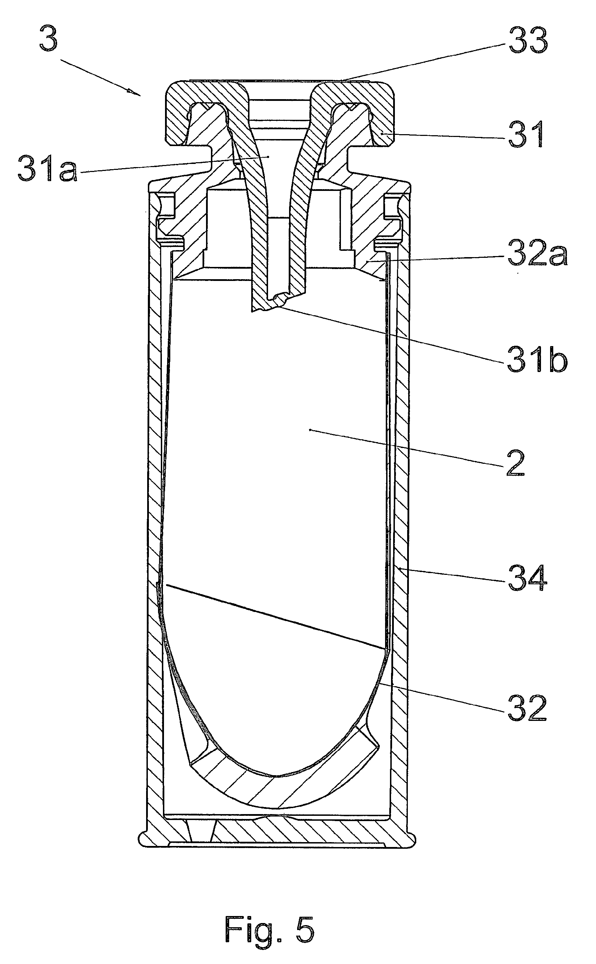

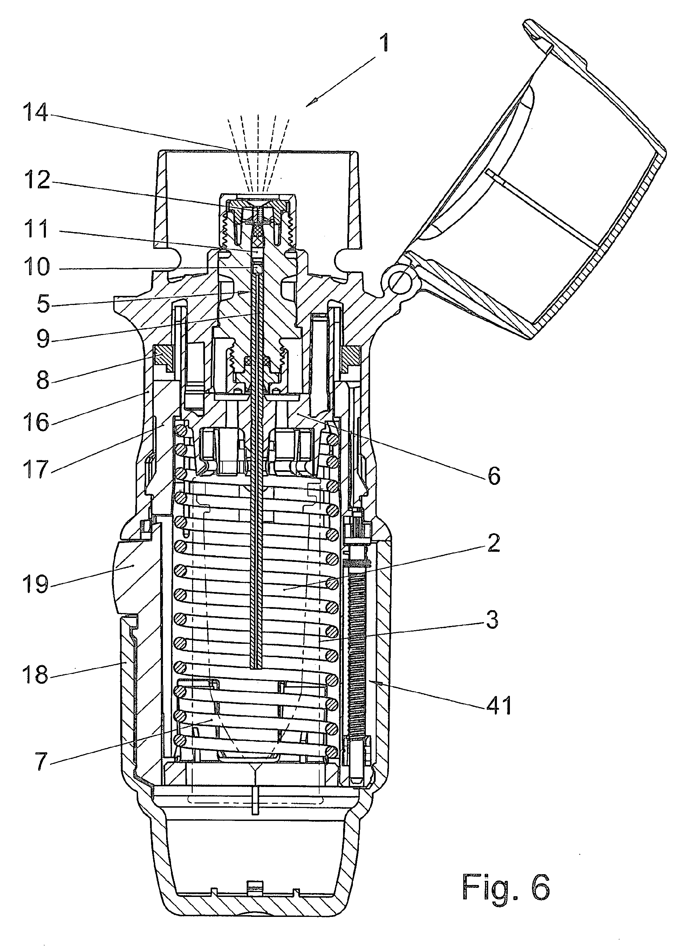

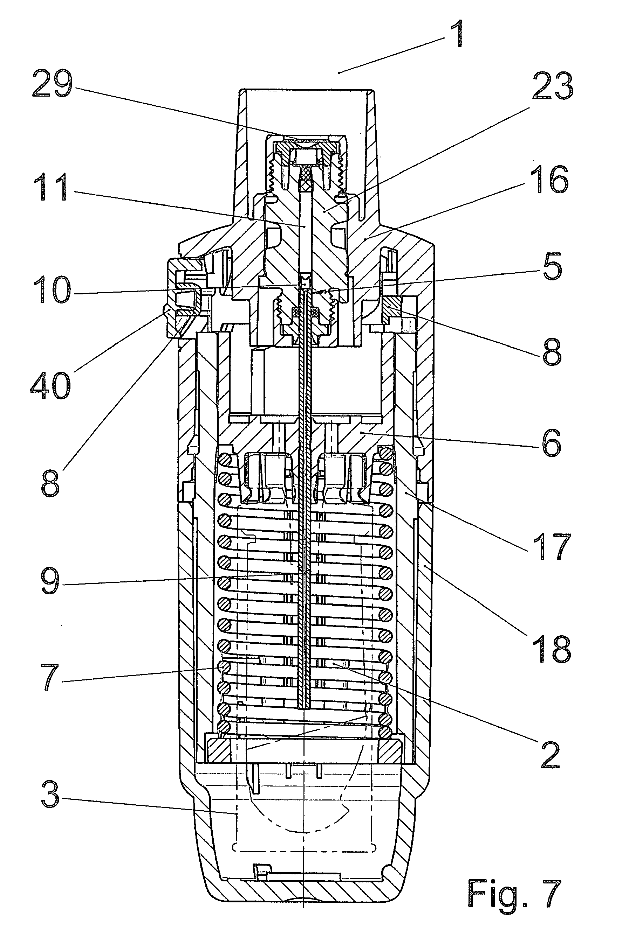

Figur 5 zeigt ein Ausführungsbeispiel eines mit einer flüssigen medizinischen Formulierung gefüllten Behälters (3), der mit dem Leckagemesssystem (100) auf Dichtigkeit geprüft werden kann.
In diesem Ausführungsbeispiel beinhaltet der Behälter (3) einen die Flüssigkeit (2) aufnehmenden Beutel (32), dessen Wandmaterial flexibel ist und beispielsweise aus einer mehrschichtig aufgebaute Folie oder desgleichen besteht, die neben einer mit der medizinischen Flüssigkeit kompatiblen Kunststoffschicht eine Metallschicht wie z.B. eine Aluminiumschicht oder ähnliches enthält, um die Diffusion oder Permeation von Gas durch die Behälterwand hindurch zu minimieren.
Insgesamt umfasst der in Figur 5 gezeigte Behälter (3) den flexiblen, unten geschlossenen, die Flüssigkeit (2) enthaltenden Beutel (32), der im oberen Bereich direkt mit einem Halt gebenden Flansch (32a) vorzugsweise aus Kunststoff verbunden ist, eine Behälterkappe (31), eine den Beutel (32) umgebenden Hülse (34) und ein optionales Siegel (33). Die Hülse (34) schützt den Beutel (32) vor mechanischen Beschädigungen von außen. Die Behälterkappe (31) ist vorzugsweise aus Kunststoff, besonders bevorzugt aus HD-PE, und insbesondere aus einem ähnlichen oder dem gleichen Material wie der Flansch (32a) und wird nach Befüllen des Beutels (32) mit Flüssigkeit (2) vorzugsweise über einen Warmverformungsprozess oder einen Schweißprozess (z.B. Ultraschall- oder Laserschweißen) dicht mit dem Flansch (32a) verbunden. Die Behälterkappe (31) weist einen in den Innenraum des Beutels (32) hinein ragenden Einführtrichter (31a) auf. Bei Anschluss des Behälters (3) an ein Gerät oder ein Werkzeug zur Ausbringung der Flüssigkeit (2), beispielsweise an einen Zerstäuber (1) wie er in Figur 6 und 7 dargestellt ist, bildet dieser Einführtrichter (31 a) eine zentrierte Führung für ein Flüssigkeits-Entnahmewerkzeug wie beispielsweise eine Hohlnadel oder Kapillare oder rohrförmige Struktur. So wird ein bezogen auf die Verbindungsstelle unkontrolliertes Anstechen des Behälters (3) durch das Entnahmewerkzeug verhindert. Vor der Anbindung an ein Entnahmewerkzeug oder ein Gerät ist der Behälter bzw. das dem Innenraum des Behälters (3) zugewandte Ende des Einführtrichters (31a) mit einer Membran (31b) verschlossen, die beim Einführen des Entnahmewerkzeugs durchstochen oder aufgeklappt wird. Auf diese Weise schützt die Membran (31b) den nicht-angestochenen Behälter vor Flüssigkeitsaustritt. Optional kann der Behälter (3) während der Lagerung zusätzlich mit einem Siegel (33) versehen sein, das beispielsweise aus einer Metallfolie vorzugsweise aus Aluminium besteht und das obere offene Ende des Einführtrichters (31 a) verschließt. Ein solches Siegel kann als Originalitätszeichen dienen und den Einführtrichter (31 a) während des Transportes des vereinzelten Behälters (3) vor Verunreinigungen schützen. Gase, welche die Membran (31b) gegebenenfalls durchdringen können, werden von einem metallischen Kopfsiegel verstärkt zurückgehalten.

Vorzugsweise dient der Behälter (3) der mehrfachen Ausbringung von Flüssigkeitseinheiten. Daher ist er vorzugsweise so beschaffen, dass der Innendruck auch bei Flüssigkeitsentnahme und angeschlossenem Entnahmewerkzeug im Wesentlichen unverändert bleibt, so dass bei Ansaugvorgängen immer die gleiche Menge Flüssigkeit (2) entnommen wird. Durch die Flexibilität des Beutels (32) oder alternativ durch eine flexible Behälterwand, die sich bei Flüssigkeitsentnahme zumindest teilweise ins Behälterinnere verschiebt, wird der Innendruck im Behälter (3) bei Flüssigkeitsentnahme durch Verkleinerung des Innenvolumens konstant gehalten. Bevorzugt werden für solche Entnahmesituationen Behälter (3), in denen die flexible Wand durch einen Beutel (32) oder auch Innenbeutel oder Folienbeutel oder Schlauch gebildet wird, der im Wesentlichen verformbar, zusammendrückbar und/oder zusammenziehbar, oder kollabierbar ausgebildet ist. Solche Behälter, wie sie auch im Leckagemesssystem (100) geprüft und deren Verdunstungsraten anhand von Prüflecks (300) im erfindungsgemäßen Verfahren nachgestellt werden können, sind beispielsweise in verschiedenen Ausführungsformen in den Schriften WO00/49988A2, WO01/076849A1, WO99/43571A1, WO09/115200A1 und WO09/103510A1 beschrieben.

Für die Anwendung mit medizinischen Geräten, wie sie anhand von Figuren 6 und 7 näher beschrieben werden, wird solch ein Behälter (3) bevorzugt, in dem sich die Flüssigkeit (2) in einem flexiblem, verformbaren und/oder kollabierbaren Beutel (32) oder Schlauch befindet. Auf diese Weise kann der Innendruck im Behälter (3) bei Flüssigkeitsentnahme konstant gehalten werden, ohne dass ein regelmäßiger Gasaustausch mit der Umgebung des Zerstäubers (1) stattfindet. Alternativ kann auch ein Behälter mit starrer Behälterwand in medizinischen Geräten eingesetzt, wobei der Innendruck dieses Behälters über eine Belüftung konstant gehalten wird. Beispielsweise wird ein solcher starrer Behälter, wie er ebenfalls zur Prüfung im Leckagemesssystem (100) oder anstelle des zu prüfenden Gegenstandes im erfindungsgemäßen Verfahren eingesetzt werden kann, in der WO06/136426A1 beschrieben.

Der hier dargestellte Behälter (3) dient der Lagerung oder Aufbewahrung einer flüssigen medizinischen Formulierung und dem Einsatz ein einem medizinischen Gerät, mit dem die Formulierung in bestimmter Weise ausgebracht wird. Der Behälter eignet sich für die Aufbewahrung sowohl wässriger als auch bevorzugt alkoholischer, insbesondere ethanolischer, medizinischer Formulierungen. Insbesondere wird hier eine zu verabreichende flüssige medizinische Formulierung eingesetzt, die eine Substanz mit geringem Dampfdruck oder eine alkoholische Verbindung enthält.

Bevorzugte Inhaltsstoffe der vorzugsweise flüssigen medizinischen Formulierung sind insbesondere in den Schriften WO09/047173A2 und WO09/115200A1 aufgeführt, in denen die angegebenen Stofflisten und Formulierungsrezepturen (WO09/115200A1 Seite 25 bis 40 und WO09/047173A2 Seite 15 bis 21) hiermit vollumfänglich als Referenz eingeführt werden. Insbesondere kann es sich bei dem in diesen Schriften beschriebenen Fluid um wässrige oder nicht wässrige Lösungen, Mischungen, Formulierungen mit und ohne Lösungsmittel, wie Ethanol oder dergleichen, handeln.

Fig. 6 und 7 zeigen in einer schematischen Darstellung ein tragbares, handbetätigtes medizinisches Gerät, das eine flüssige medizinische Formulierung enthält und dessen Dichtigkeit mit dem Leckagemesssystem (100) untersucht werden kann. Bei dem Gerät aus Fig. 6 und 7 handelt es sich um einen treibgasfreien Zerstäuber (1), der mittels einer rein mechanischen Hochdruckpumpe pro Betätigungszyklus die jeweilig vorbestimmte Menge einer Flüssigkeit (2) bzw. einer flüssigen medizinischen Formulierung als vorzugsweise lungengängiges bzw. inhalierfähiges Aerosol (14) aus einer geeigneten Düse ausgibt. Dieses Aerosol (14) mit Tröpfchen mit aerodynamischen Durchmessern von vorzugsweise 3 bis 10 Mikrometern kann von einem nicht dargestellten Benutzer eingeatmet werden.

Beim Betrieb des Zerstäubers wird unterschieden zwischen dem ungespannten Zustand mit unbefülltem Dosiervolumen in der Druckkammer (11) (Fig. 6) und dem gespannten Zustand mit befüllter Druckkammer (11) (Fig. 7).

Beim Spannen des Zerstäubers (1) wird sein Gehäuseoberteil (16) relativ zum Gehäuseinnenteil (17) und Gehäuseunterteil (18) um einen festen Drehwinkel von z.B. 180° gedreht. Mittels eines innen angeordneten Schraubgetriebes wird durch die Relativdrehung eine Kolbenpumpe angetrieben. Insbesondere wird dabei eine Halterung (6) verschoben, die fest mit dem Hohlkolben (9) verbunden ist, der in das die Druckkammer (11) bildende Zentralteil (23) hineinragt. Auf der dem Zentralteil (23) abgewandten Seite der Halterung (6) ist der die Flüssigkeit (2) beinhaltende Behälter (3) angeschlossen und der Hohlkolben (9) in seinen Inhalt eintaucht. Der Hohlkolben (9) erfüllt hierbei auch die Funktion eines Entnahmewerkzeugs für die Flüssigkeit (2) aus dem Behälter (3). Wird nun die Halterung (6) so verschoben, dass sich der Hohlkolben stückweit aus dem Zentralteil (23) zurückzieht, wird eine vorbestimmte, ggf. einstellbare Menge der Flüssigkeit (2) aus dem Behälter (3) in die Druckkammer gefördert und gleichzeitig die Antriebsfeder (7) des Druckerzeugers (5) gespannt (Endzustand des Spannvorgangs ist in Fig. 7 dargestellt). Beim Auslösen des Zerstäubers (1), d.h. bei Betätigung eines Sperrrings (8) über eine Taste (40) wird die in der Antriebsfeder (7) gespeicherte Energie des Druckerzeugers (5) freigesetzt: Der zuvor zur Flüssigkeitsförderung benutzte Hohlkolben (9), der gleichzeitig Teil der Hochdruckpumpe des Geräts ist, drückt nun mit geschlossenem Rückschlagventil (10) in die Druckkammer (11), so dass die durch die Hubbewegung des Hohlkolben (9) vorbestimmte Flüssigkeitsmenge von dort durch die Düse (12) ausgebracht wird. Das Gerät befindet sich nun wieder im entspannten Zustand (Fig. 6).

Je nach Variante des Zerstäubers (1) kann die Halterung (6) so ausgeformt sein, dass der Behälter (3) austauschbar ist oder nicht. Im Falle eines austauschbaren Behälters (3) weist das Gehäuseinnenteil (17) des Zerstäubers (1) vorzugsweise einen Sicherheitsverschluss (19) auf, der ein versehentliches Öffnen des Zerstäubers (1) bzw. Abziehen des Gehäuseunterteils (18) verhindert. Insbesondere muss zum Lösen des Gehäuseunterteils (18) der Sicherheitsverschluss (19) gegen eine Federkraft eingedrückt werden.

In einer Variante kann der Zerstäuber (1) auch so gestaltet sein, dass der Behälter (3) im Zerstäuber (1) vorinstalliert ist.

Der hier dargestellte Zerstäuber (1) ist nur eines von vielen möglichen medizinischen Handgeräten, die mit dem Leckagemesssystem (100) auf Dichtigkeit geprüft werden können. Anhand dieses Beispiels ist leicht zu erkennen, wie andere flüssigkeits-beinhaltende Geräte, so lange sie ähnlich handliche Größen haben, ebenfalls in die Testkammer (101) eines Leckagemesssystems (100) eingesetzt werden können. Tauscht man die der Zerstäubung dienende Düse (12) oder Düsenbaugruppe (29) dieses Geräts beispielsweise gegen den Kopf eines Flüssigkeitsspenders oder gegen eine Injektionsdüse bzw. eine Kanüle oder sonstige Einspritzvorrichtung aus, so erhält man Spendersysteme oder Injektoren, die direkt als Ausführungsbeispiele für Gerätegruppen angegeben werden können, deren Dichtigkeit ebenfalls mit dem Leckagemessystem (100) untersucht werden kann.

Vorzugsweise sind die hier betrachteten medizinischen Handgeräte auf die Ausbringung mehrerer Dosiseinheiten einer flüssigen, medizinischen Formulierung ausgelegt. So zeigt der Zerstäuber (1) in Figur 5 ein Zählwerk (41), beispielsweise ein durch die Gehäusedrehung angetriebenes Spindel-Reiter-Zählwerk, anhand dessen der nicht dargestellte Benutzer die Menge der entnommenen oder der im Gerät verbleibenden Dosiseinheiten ablesen kann. Der in das Gerät eingesetzte Behälter enthält eine Menge an Flüssigkeit (2), die für die Entnahme mehrerer Dosiseinheiten ausreicht. Im konkreten Darstellungsbeispiel enthält der Behälter (3) eine Menge an Flüssigkeit (2), die für die Ausbringung von vorzugsweise 30 bis 180 Dosiseinheiten ausreicht. Beispielsweise enthält der Behälter (3) 2 bis 10 Milliliter der Flüssigkeit (2).

Der hier dargestellte Zerstäuber (1) dient lediglich als ein einziges Beispiel aus der Vielzahl an flüssigkeits-enthaltenden Geräten, die in das Leckagemesssystem (100) eingesetzt, oder durch Prüflecks im erfindungsgemäßen Verfahren nachgestellt werden können. Weitere Beispiele dieser Art sind aus dem Bereich der medizinischen Handgeräte in Form von Injektoren und in Form von weiteren Zerstäubern für unterschiedliche Anwendungen in den Schriften WO97/12687A1, WO2004/024340A1, WO2007/128381A1, WO2005/080001A1, WO2009/047173A2, WO2005/107837A1, WO2004/022244A1, WO2006/125577A2, WO2009103510A1, WO03/002045A1 und US2004/0015126A1 beschrieben.

**Bezugszeichenliste**

| | | | |
|---|---|---|---|
| 1 | Zerstäuber | 100 | Leckagemesssystem |
| 2 | Flüssigkeit | 101 | Testkammer |
| 3 | Behälter | 102 | Sensor |
| 5 | Druckerzeuger | 103 | Bypass |
| 6 | Halterung | 104 | Pumpe |
| 7 | Antriebsfeder | 105 | Dreiwegeventil |
| 8 | Sperring | 106 | Waschflasche |
| 9 | Hohlkolben | 107 | Heizung |
| 10 | Rückschlagventil | 108 | Lufteinlass |
| 11 | Druckkammer | 109 | Luflauslass |
| 12 | Düse | 110 | Einlass (für Trägergas) |
| 14 | Aerosol | 111 | Gasauslass |
| 16 | Gehäuseoberteil | | |
| 17 | Gehäuseinnenteil | | |
| 18 | Gehäuseunterteil | 300 | Prüfleck |
| 19 | Sicherheitsverschluss | 301 | Speichermaterial |
| 23 | Zentralteil | 302 | Gefäß |
| 29 | Düsenbaugruppe | 303 | Folie |
| 31 | Behälterkappe | 304 | Deckel |
| 31a | Einführtrichter(inBehälterkappe) | 304a | Bohrung (im Deckel) |
| 31b | Membran (in Behälterkappe) | 305 | Stützscheibe |
| 32 | Beutel | | |
| 32a | Flansch (am Beutel) | | |
| 33 | Siegel | | |
| 34 | Hülse | | |
| | | | |
| 40 | Taste | | |
| 41 | Zählwerk | | |

## Patentansprüche

1. Prüfleck (300),
- das eine Flüssigkeit enthält und
- das eine als Verdunstungsbarriere dienende Festkörperschicht beinhaltet, welche die Permeation von aus der Flüssigkeit gebildetem Gas oder Dampf oder von Molekülen aus der Flüssigkeit durch die Festkörperschicht hindurch zulässt, und
- aus dem Gas oder Dampf oder von Gas transportierte
Flüssigkeitsbestandteile austreten,
zum Einsatz in einem gasanalytischen Messsystem oder Leckagemesssystem (100) oder zur Überprüfung eines gasanalytischen Messsystems oder Leckagemesssystems (100), wobei
das aus dem Prüfleck (300) austretende Gas oder der austretende Dampf oder
die von Gas transportierten austretenden Flüssigkeitsbestandteile von der Flüssigkeit durch den ihr eigenen Dampfdruck gebildet werden, **dadurch gekennzeichnet dass** die Flüssigkeit in einem Speichermaterial (301) eingelagert oder gebunden oder tamponiert ist und die Flüssigkeit keinen direkten Kontakt zu dem Bereich der Festkörperschicht aufweist, durch den Gas oder Dampf oder Flüssigkeitsbestandteile aus dem Prüfleck (300) austreten können.

2. Prüfleck (300) nach Anspruch 1 **dadurch gekennzeichnet, dass** die Festkörperschicht eine Membran oder Folie (303) ist, an der das Gas oder der Dampf aus dem Prüfleck (300) austritt.

3. Prüfleck (300) nach Anspruch 1 oder 2 **dadurch gekennzeichnet, dass** das Prüfleck den Austritt von Gas, Dampf oder Flüssigkeitsbestandteilen nur an einem für die Permeation vorgesehenen Bereich der Festkörperschicht oder nur an einer Permeationsöffnung zulässt und dass das Prüfleck ansonsten überall dicht ist und insbesondere keine Flüssigkeit austritt.

4. Prüfleck (300) nach einem der vorherigen Ansprüche **dadurch gekennzeichnet,**
**dass** an der dem Inneren des Prüflecks zugewandten Oberfläche der Festkörperschicht ein Gas gefüllter Raum angrenzt, wobei das Gas dem aus dem Prüfleck (300) austretenden Gas entspricht oder in Form einer Mischung mit diesem gesättigt ist.

5. Prüfleck (300) nach einem der vorherigen Ansprüche **dadurch gekennzeichnet, dass** das Speichermaterial (301) ein Schwamm oder Filtermaterial ist.

6. Prüfleck (300) nach einem der Ansprüche 2 bis 5 **dadurch gekennzeichnet, dass** die Festkörperschicht eine Folie (303) oder Polymerfolie ist, die insbesondere aus PE, PP, Polystyrol oder Acetal hergestellt ist.

7. Prüfleck (300) nach einem der Ansprüche 2 bis 6 **dadurch gekennzeichnet, dass** die Festkörperschicht eine Dicke von 20 Mikrometern bis ein Millimeter hat.

8. Prüfleck (300) nach einem der vorherigen Ansprüche **dadurch gekennzeichnet, dass** das Prüfleck (300) eine als Verdunstungsbarriere dienende, insbesondere von einer Folie (303) gebildete Festkörperschicht, ein Gefäß (302) insbesondere aus Metall oder Glas und einen insbesondere mit einer Bohrung oder Durchlass ausgestatteten Deckel (304) beinhaltet oder dass das Prüfleck (300) eine Membran oder Folie (303), ein Gefäß (302), einen Deckel (304) und einen Stützring oder eine Stützscheibe (305) umfasst.

9. Prüfleck (300) nach Anspruch 8 **dadurch gekennzeichnet, dass** der Deckel (304) durch eine Crimpung oder eine Verschraubung an dem Gefäß (302) befestigt ist.

10. Prüfleck (300) nach einem der vorherigen Ansprüche **dadurch gekennzeichnet, dass** das Prüfleck (300) eine Heizung oder eine Temperaturregelung beinhaltet.

11. Prüfleck (300) nach einem der vorherigen Ansprüche **dadurch gekennzeichnet, dass** die Flüssigkeit ein Lösemittel, bevorzugt ein in medizinischen Formulierungen einsetzbares Lösemittel, besonders bevorzugt Wasser oder eine alkoholische Verbindung wie Ethanol ist.

12. Verfahren zur Überprüfung oder Kalibrierung eines gasanalytischen Messsystems oder eines Leckagemesssystems (100), **dadurch gekennzeichnet dass** ein Prüfleck (300) gemäß Anspruch 1 zur Signalerzeugung oder zum Test auf das Vorhandensein eines Signals im Messsystem verwendet wird oder mehrere unterschiedlich ausgasende Prüflecks (300) gemäß Anspruch 1 zur Erfassung eines Datensatzes im Messsystem verwendet werden.

13. Verfahren nach Anspruch 12, **dadurch gekennzeichnet, dass** die Überprüfung des Leckagemesssystems (100) eine Eignungsprüfung des Leckagemesssystemen (100) für seinen Einsatz bei der Dichtigkeitsprüfung eines Flüssigkeit beinhaltenden Gegenstandes darstellt und dass hierbei ein Prüfleck (300) nach Anspruch 1 verwendet wird, wobei das Prüfleck (300) die gleiche Flüssigkeit oder das gleiche flüssige Lösemittel oder den gleichen flüchtigen Bestandteil wie der auf Dichtigkeit zu prüfende Gegenstand enthält.

14. Verfahren nach Anspruch 13 **dadurch gekennzeichnet, dass** die äußeren Abmessungen oder das äußere Gesamtvolumen des Prüflecks (300) den jeweiligen Werten des zu prüfenden Gegenstands entsprechen oder dass Abweichungen zwischen den äußeren Volumina von Prüfleck (300) und zu prüfendem Gegenstand bei der Auswertung der durch den zu prüfenden Gegenstand im Leckagemesssystem (100) erzeugten Signale in Form einer Umrechnung berücksichtigt werden.

15. Verfahren nach Anspruch 13 oder 14 **dadurch gekennzeichnet, dass** das Prüfleck (300) eine Verdunstungsrate oder eine Gasaustrittsrate in einer solchen Höhe aufweist oder in einer Testkammer (101) des Leckagemesssystems (100) eine Gaskonzentration in solcher Höhe erzeugt,
wie sie gemäß der Auslieferbedingungen des Gegenstandes für diesen nicht zulässig ist oder dem für den Gegenstand festgelegten die Grenzwert entspricht.

16. Verfahren nach einem der Ansprüche 12 bis 15 **dadurch gekennzeichnet, dass** die Verdunstungs- oder Leckagerate des Prüflecks (300) regelmäßig durch Gewichtsüberprüfung kontrolliert wird.

17. Verfahren nach einem der Ansprüche 13 bis 16 **dadurch gekennzeichnet, dass** der auf Dichtigkeit zu prüfende Gegenstand ein mit einer flüssigen medizinischen Formulierung gefüllter Behälter (3) ist.

18. Verfahren nach Anspruch 17 **dadurch gekennzeichnet, dass** der Behälter (3) eine zumindestens bereichsweise verformbare Wandung in direktem Kontakt zur Flüssigkeit (2) aufweist und oder sich die Flüssigkeit (2) im Behälter (3) in einem kollabierbaren Beutel befindet.

19. Verfahren nach einem der Ansprüche 13 bis 16 **dadurch gekennzeichnet, dass** der auf Dichtigkeit zu prüfende Gegenstand ein medizinisches Handgerät insbesondere ein Zerstäuber (1) oder Injektor ist, der eine flüssige medizinische Formulierung enthält.

## Claims

1. Test leak (300)
- which contains a liquid and
- which contains a solid layer acting as an evaporation barrier which allows gas or vapour formed from the liquid, or molecules from the liquid, to permeate through the solid layer, and
- from which gas or vapour or gas-borne liquid ingredients escape,
for use in a gas-analytical measuring system or leakage measuring system (100) or for testing a gas-analytical measuring system or leakage measuring system (100), wherein the gas or the vapour or the gas-borne liquid ingredients escaping from the test leak (300) are formed from the liquid by their own inherent vapour pressure, **characterised in that** the liquid is incorporated or bound or buffered in a storage material (301) and the liquid does not come into direct contact with the region of the solid layer through which gas or vapour or liquid ingredients are able to escape from the test leak (300).

2. Test leak (300) according to claim 1, **characterised in that** the solid layer is a membrane or film (303) at which the gas or the vapour exits from the test leak (300).

3. Test leak (300) according to claim 1 or 2, **characterised in that** the test leak allows gas, vapour or liquid ingredients to escape only at a region of the solid layer provided for permeation or only at a permeation opening, and **in that** the test leak is leaktight everywhere else and in particular no liquid escapes.

4. Test leak (300) according to one of the preceding claims, **characterised in that** a gas-filled chamber is adjacent to the surface of the solid layer facing the inside of the test leak, the gas corresponding to the gas which escapes from the test leak (300) or being saturated therewith in the form of a mixture.

5. Test leak (300) according to one of the preceding claims, **characterised in that** the storage material (301) is a sponge or filter material.

6. Test leak (300) according to one of claims 2 to 5, **characterised in that** the solid layer is a film (303) or polymer film, in particular made from PE, PP, polystyrene or acetal.

7. Test leak (300) according to one of claims 2 to 6, **characterised in that** the solid layer has a thickness of from 20 microns to one millimetre.

8. Test leak (300) according to one of the preceding claims, **characterised in that** the test leak (300) comprises a solid layer acting as an evaporation barrier and formed in particular by a film (303), a receptacle (302), particularly of metal or glass, and a cover (304) provided in particular with a bore or opening, or **in that** the test leak (300) comprises a membrane or film (303), a receptacle (302), a cover (304) and a support ring or a support disc (305).

9. Test leak (300) according to claim 8, **characterised in that** the cover (304) is secured to the receptacle (302) by a crimp or screw connection.

10. Test leak (300) according to one of the preceding claims, **characterised in that** the test leak (300) comprises a heating means or temperature regulating means.

11. Test leak (300) according to one of the preceding claims, **characterised in that** the liquid is a solvent, preferably a solvent that can be used in medicinal formulations, particularly preferably water or an alcoholic compound such as ethanol.

12. Method for testing or calibrating a gas-analytical measuring system or a leakage measuring system (100), **characterised in that** a test leak (300) according to claim 1 is used to generate a signal or to test for the presence of a signal in the measuring system, or a plurality of test leaks (300) according to claim 1 with different gas emissions are used to determine a data set in the measuring system.

13. Method according to claim 12, **characterised in that** the testing of the leakage measuring system (100) is a test of the suitability of the leakage measuring system (100) for use in the leak testing of an object containing a liquid, and a test leak (300) according to claim 1 is used for this, the test leak (300) containing the same liquid or the same liquid solvent or the same volatile ingredient as the object which is to be tested for leaks.

14. Method according to claim 13, **characterised in that** the external dimensions or the external overall volume of the test leak (300) correspond to the respective values of the object which is to be tested, or **in that** deviations between the external volumes of the test leak (300) and the object which is to be tested are taken into account, in the form of a conversion, in the evaluation of the signals generated by the object which is to be tested in the leakage measuring system (100).

15. Method according to claim 13 or 14, **characterised in that** the test leak (300) has an evaporation rate or a gas exit rate or, in a test chamber (101) of the leakage measuring system (100), generates a gas concentration of a level that is not permissible for the object, according to the delivery conditions of the object, or that corresponds to the limit value specified for the object.

16. Method according to one of claims 12 to 15, **characterised in that** the evaporation or leakage rate of the test leak (300) is monitored regularly by weight checks.

17. Method according to one of claims 13 to 16, **characterised in that** the object which is to be tested for leaks is a container (3) filled with a liquid medicinal formulation.

18. Method according to claim 17, **characterised in that** the container (3) comprises a wall which is deformable at least in parts and is in direct contact with the liquid (2) and/or the liquid (2) in the container (3) is held in a collapsible bag.

19. Method according to one of claims 13 to 16, **characterised in that** the object which is to be tested for leaks is a hand-held medical device, particularly a nebuliser (1) or injector, which contains a liquid medicinal formulation.

## Revendications

1. Fuite d'essai (300),
- qui contient un liquide et
- qui comporte une couche de corps solide faisant office de barrière à l'évaporation, laquelle permet la perméation d'un gaz ou d'une vapeur formé(e) à partir du liquide ou de molécules issues du liquide à travers la couche de corps solide, et
- de laquelle proviennent le gaz ou la vapeur ou des composantes de liquide transportées par le gaz,
destinée à être utilisée dans un système de mesure ou un système de mesure de fuites (100) d'analyse de gaz ou destinée à surveiller un système de mesure ou un système de mesure de fuites (100) d'analyse de gaz, le gaz ou la vapeur provenant de la fuite d'essai (300) ou les composantes de liquide transportées provenant du gaz étant formés par le liquide du fait de la pression de vapeur qui lui est propre, **caractérisée en ce que** le liquide est stocké ou tamponné dans un matériau accumulateur (301) ou est lié à ce dernier, et **en ce que** le liquide ne présente aucun contact direct avec la zone de la couche de corps solide, par laquelle du gaz ou de la vapeur ou des composants de liquide peuvent provenir de la fuite d'essai (300).

2. Fuite d'essai (300) selon la revendication 1, **caractérisée en ce que** la couche de corps solide est une membrane ou un film (303), au niveau de laquelle/duquel le gaz ou la vapeur proviennent de la fuite d'essai (300).

3. Fuite d'essai (300) selon la revendication 1 ou 2, **caractérisée en ce que** la fuite d'essai permet la sortie de gaz, de vapeur ou de composants de liquide uniquement au niveau d'une zone, prévue pour la perméation, de la couche de corps solide ou uniquement au niveau d'une ouverture de perméation, et **en ce que** la fuite d'essai est sinon partout ailleurs étanche et qu'en particulier aucun liquide ne sort.

4. Fuite d'essai (300) selon l'une quelconque des revendications précédentes, **caractérisée en ce qu'**à l'intérieur de la fuite d'essai tournée vers la surface de la couche de corps solide, est adjacent un espace rempli de gaz, où le gaz provenant de la fuite d'essai (300) est égal au gaz de sortie, ou est suturé avec ce dernier sous la forme d'un mélange.

5. Fuite d'essai (300) selon l'une quelconque des revendications précédentes, **caractérisée en ce que** le matériau accumulateur (301) est une éponge ou un matériau filtrant.

6. Fuite d'essai (300) selon l'une quelconque des revendications 2 à 5, **caractérisée en ce que** la couche de corps solide est un film (303) ou un film polymère, qui est fabriqué en particulier à partir de PE, de PP, de polystyrène ou d'acétal.

7. Fuite d'essai (300) selon l'une quelconque des revendications 2 à 6, **caractérisée en ce que** la couche de corps solide présente une épaisseur allant de 20 micromètres à un millimètre.

8. Fuite d'essai (300) selon l'une quelconque des revendications précédentes, **caractérisée en ce que** la fuite d'essai (300) comporte une couche de corps solide faisant office de barrière à l'évaporation, formée en particulier par un film (303), un récipient (302) en particulier en métal ou en verre et un couvercle (304) doté en particulier d'un alésage ou d'un passage, ou **en ce que** la fuite d'essai (300) comprend une membrane ou un film (303), un récipient (302), un couvercle (304) et une bague de support ou une rondelle de support (305).

9. Fuite d'essai (300) selon la revendication 8, **caractérisée en ce que** le couvercle (304) est fixé au niveau du récipient (302) par un sertissage ou un vissage.

10. Fuite d'essai (300) selon l'une quelconque des revendications précédentes, **caractérisée en ce que** la fuite d'essai (300) comporte un chauffage ou un système de régulation de température.

11. Fuite d'essai (300) selon l'une quelconque des revendications précédentes, **caractérisée en ce que** le liquide est un dissolvant, de préférence un dissolvant pouvant être utilisé dans des formulations médicales, de manière particulièrement préférée de l'eau ou une composition à base d'alcool telle que l'éthanol.

12. Procédé servant à surveiller ou à calibrer un système de mesure ou un système de mesure de fuites (100) à analyse de gaz, **caractérisé en ce qu'**une fuite d'essai (300) selon la revendication 1 est utilisée pour produire un signal ou pour tester la présence d'un signal dans le système de mesure, ou **en ce que** plusieurs diverses fuites d'essai (300) à dégagement de gaz selon la revendication 1 sont utilisées pour détecter un jeu de données dans le système de mesure.

13. Procédé selon la revendication 12, **caractérisé en ce que** la vérification du système de mesure de fuites (100) constitue un contrôle propre du système de mesure de fuites (100) pour son utilisation lors du contrôle d'étanchéité d'un objet comportant un liquide, et **en ce qu'**à cet effet une fuite d'essai (300) selon la revendication 1 est utilisée, la fuite d'essai (300) contenant le même liquide ou le même dissolvant liquide ou le même composant volatile que l'objet dont il convient de contrôler l'étanchéité.

14. Procédé selon la revendication 13, **caractérisé en ce que** les dimensions extérieures ou le volume global extérieur de la fuite d'essai (300) correspondent aux valeurs respectives de l'objet à contrôler, ou **en ce que**
des écarts entre les volumes extérieurs de la fuite d'essai (300) et l'objet à contrôler sont pris en compte lors de l'analyse des signaux produits par l'objet à contrôler dans le système de mesure de fuites (100) sous la forme d'une conversion.

15. Procédé selon la revendication 13 ou 14, **caractérisé en ce que** la fuite d'essai (300) présente un taux d'évaporation ou un taux de sortie de gaz d'un niveau du même type ou produit dans une chambre de test (101) du système de mesure de fuites (100) une concentration de gaz d'un niveau du même type que le niveau non admissible selon les conditions de livraison de l'objet pour ce dernier ou correspondant à la valeur limite fixée pour l'objet.

16. Procédé selon l'une quelconque des revendications 12 à 15, **caractérisé en ce que** les taux d'évaporation ou de fuite de la fuite d'essai (300) sont régulièrement contrôlés par la vérification du poids.

17. Procédé selon l'une quelconque des revendications 13 à 16, **caractérisé en ce que** l'objet dont il convient de vérifier l'étanchéité est un récipient (3) rempli d'une formulation médicale liquide.

18. Procédé selon la revendication 17, **caractérisé en ce que** le récipient (3) présente une paroi déformable au moins par endroits en contact direct avec le liquide (2), et/ou **en ce que** le liquide (2) se trouve dans le récipient (3) dans un sachet repliable.

19. Procédé selon l'une quelconque des revendications 13 à 16, **caractérisé en ce que** l'objet dont il convient de vérifier l'étanchéité est un appareil médical portatif en particulier un atomiseur (1) ou un injecteur, qui contient une formulation médicale liquide.
